(19) Europäisches Patentamt
European Patent Office
Office européen des brevets

(11) **EP 3 222 711 B1**

(12) **EUROPEAN PATENT SPECIFICATION**

(45) Date of publication and mention
of the grant of the patent:
**06.01.2021 Bulletin 2021/01**

(21) Application number: **15860074.2**

(22) Date of filing: **21.11.2015**

(51) Int Cl.:
*C12N 5/00* (2006.01)  *C12N 5/07* (2010.01)
*A61F 2/00* (2006.01)  *A61F 2/02* (2006.01)
*A61L 27/04* (2006.01)  *A61L 27/34* (2006.01)
*A61L 27/50* (2006.01)

(86) International application number:
**PCT/ES2015/070836**

(87) International publication number:
**WO 2016/079366 (26.05.2016 Gazette 2016/21)**

(54) **PRODUCTION OF ARTIFICIAL TISSUES COMPRISING MAGNETIC PARTICLES**

HERSTELLUNG VON KÜNSTLICHEM GEWEBE MIT MAGNETTEILCHEN

ÉLABORATION DE TISSUS ARTIFICIELS COMPRENANT DES PARTICULES MAGNÉTIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.11.2014 ES 201400958**

(43) Date of publication of application:
**27.09.2017 Bulletin 2017/39**

(73) Proprietors:
• **Universidad de Granada**
 **E-18071 Granada (ES)**
• **Servicio Andaluz de Salud**
 **41071 Sevilla (ES)**
• **Universidad Nacional de Córdoba**
 **Córdoba, 5000 (AR)**

(72) Inventors:
• **LÓPEZ LÓPEZ, Modesto Torcuato**
 **18071 Granada (ES)**
• **GARCÍA LÓPEZ-DURÁN, Juan de Dios**
 **18071 Granada (ES)**
• **ALAMINOS MINGORANCE, Miguel**
 **18071 Granada (ES)**
• **RODRÍGUEZ, Ismael Ángel**
 **5000 Córdoba (AR)**
• **SCIONTI, Giuseppe**
 **18014 Granada (ES)**

(74) Representative: **Hoffmann Eitle**
 **Hoffmann Eitle S.L.U.**
 **Paseo de la Castellana 140, 3a planta**
 **Edificio LIMA**
 **28046 Madrid (ES)**

(56) References cited:
**EP-A2- 2 471 902**  **WO-A1-2013/190165**
**US-A1- 2004 147 015**  **US-A1- 2006 093 611**
**US-A1- 2011 034 753**  **US-A1- 2011 085 968**
**US-B2- 7 811 297**

**Description**

The present invention is comprised in the field of biomedicine, and more specifically the field of tissue engineering. The invention specifically relates to an *in vitro* method for producing an artificial tissue using magnetic particles, to the artificial tissue that can be produced by said method, and to the use of said artificial tissue for partially or completely increasing, restoring or replacing the functional activity of a damaged organ or tissue.

**PRIOR ART**

Tissue engineering constitutes a set of techniques and disciplines that allows designing and generating artificial tissues in a laboratory using cells from tissue samples obtained from biopsies, and is therefore an enormous breakthrough in organ transplant and regenerative medicine. Tissue engineering is one of the biotechnology areas that has undergone major development in recent years due to its potential usefulness in the *in vitro* production of tissues and organs for implant in patients in need of these tissues. Nevertheless, the artificial tissues described up until now present a number of problems and complications; some of which are explained below, using as an example, artificial skin, cornea, bladder and urethra tissues.

Tissue engineering is an interdisciplinary field that applies the principles of biology and engineering to develop strategies targeting the replacement, repair, maintenance and/or improvement of biological tissues [Langer R, Vacanti JP. Tissue engineering. Science 260 (5110), 920-926 (1993)]. Three different tissue engineering approaches can be discerned: (i) exclusive use of cells; (ii) exclusive use of polymer matrices; and (iii) a cell and polymer matrix combination [Baddour JA, et al. Birth Defects Res. Part C-Embryo Today-Rev. 96, 1-29 (2012); Khademhosseini A, et al. Proc. Natl. Acad. Sci. U.S.A. 103, 2480-2487 (2006)].

The extracellular matrix (polymer matrix in artificial biomaterial) also plays an essential role as it provides the support required for cell proliferation and cell main function maintenance. An ideal polymer matrix must have the following characteristics [Hutmacher DW. Biomaterials 21, 2529-2543 (2000)]: (i) it must be three-dimensional and highly porous, with interconnected pores to facilitate cell growth and nutrient transport; (ii) it must have surface chemical properties suitable for allowing cell adhesion, proliferation and differentiation; (iii) it must have biomechanical properties suited to the native tissue to be replaced. This last characteristic is particularly important in the case of load-bearing tissues, such as tissues from the musculoskeletal system, such as cartilage and bone. By way of example, it must be taken into account that only 3-5% of cartilage volume is made up of cells (chondrocytes), which in turn have moduli of mechanical rigidity that is several orders of magnitude higher than those of the extracellular matrix, so they do not significantly contribute to the mechanical properties of cartilage as a whole [Han L, et al. Ann. Rev. Mater. Res. 41, 133-168 (2011)].

The materials that are used in tissue engineering to generate the polymer matrix can be classified as natural and synthetic materials. Included among natural materials are collagen, fibrin, alginate, agarose, chitosan and hyaluronic acid. They have the advantage of being biocompatible and biodegradable, and of providing physiological conditions suitable for cell adhesion and proliferation [Pabbruwe MB, et al. Biomaterials 30, 4277-4286 (2009)]. However, they all they have the drawback of being mechanically weak, such that the cellular matrices generated by means of these polymers do not have biomechanical properties suitable for the efficient use thereof in the majority of possible applications in tissue engineering. In contrast, synthetic materials, such as those based on the alpha-hydroxy acids, have readily moldable mechanical properties, but they have the important drawback of no having natural sites for cell adhesion.

An alternative to the use of natural or synthetic materials is the use of composites made of these types of materials. For example, Kon E, et al. [Injury 41, 693-701 (2010)] prepared an artificial polymer matrix from collagen fibers and hydroxyapatite nanoparticles for the purpose of reproducing parts of cartilage. Along the same lines, different authors have very recently proposed the use of magnetic nanoparticles combined with polymers to produce magnetic polymer matrices for replacing tissues [Bock N, et al. Acta Biomater. 6, 786-796 (2010); Das B, et al. Biomed. Mater. 8, 035003 (2013); Hu H, et al. RSC Adv. 3, 879-886 (2013); Li, et al. Adv. Funct. Mater. 23, 660-672 (2013); Zhu, et al. J. Mater. Chem. B 1, 1279-1288 (2013)].

EP 2 471 902 discloses: An in vitro method for preparing an artificial tissue comprising: a)adding a composition comprising fibrinogen to a sample of isolated cells, b)adding an antifibrinolytic agent to the product resulting from step (a), c)adding at least one coagulation factor, a source of calcium, thrombin, or any combination of the above to the product resulting from step (b), d)adding a composition of a polysaccharide to the product resulting from step (c), e)culturing isolated cells in or on the product resulting from step (d), and f)inducing the nanostructuring of the product resulting from step (e).

Many of the artificial tissues produced by tissue engineering, and particularly those produced by means of fibrin and agarose, have mechanical properties (viscoelastic moduli) that are too weak to be implanted as replacements for most native human tissues. The introduction of magnetic particles in artificial tissues would allow increasing their mechanical strength. However, the magnetic matrices referred to in earlier papers do not have mechanical properties that are dependent in the strength of a possible magnetic field that is applied.

Furthermore, the magnetic particles used in earlier papers are of a size in the order of 10 nm, and therefore present certain drawbacks since they can be endocytosed, therefore presenting a higher risk of toxicity, whereas larger sized particles can only be phagocytosed, and would therefore only affect the macrophages. Said particles are single magnetic domain particles. This means that the force of interaction between them is very weak, lower than the elastic force of the polymer matrix and Brownian motion, such that the application of a magnetic field does not modify the internal structure of the biomaterial.

The tissue engineering is one of the fastest growing areas in biotechnology. However, the drawbacks of artificial tissues existing up until now call for the need to develop new techniques that allow producing artificial tissues that can be used in human clinical practice or for the evaluation of pharmacological and chemical products, exceeding those limitations detected up until now.

## SUMMARY OF THE INVENTION

A **first aspect** of the invention relates to an artificial tissue, hereinafter *"artificial tissue of the invention*," as defined in claim 1.

A **second aspect** of the invention relates to the use of the artificial tissue of the invention for the evaluation of a pharmacological and/or chemical product.

A **third aspect** of the disclosure relates to the use of the artificial tissue of the invention for partially or completely increasing, restoring or replacing the functional activity of a diseased or damaged tissue or organ.

It also relates to the use of the artificial tissue of the invention for the production of a drug.

An **fourth aspect** of the invention relates to a pharmaceutical composition comprising the artificial tissue of the invention.

A preferred embodiment of this fourth aspect of the invention relates to a pharmaceutical composition comprising the artificial tissue of the invention for partially or completely increasing, restoring or replacing the functional activity of a diseased or damaged tissue or organ.

A **fifth aspect** of the invention relates to a kit or device, hereinafter *"kit or device of the invention*," comprising:

a) the artificial tissue of the invention, and
b) an element capable of generating a magnetic field.

## BRIEF DESCRIPTION OF THE FIGURES

**Figure 1.** Phase-contrast microscopy image of human fibroblasts in liquid solution. **A)** Positive control cells, having a spindle shape that is typical of fibroblasts. **B** and **C)** Cells cultured in the presence of MagNP-OH nanoparticles at concentrations of 0.5 and 1% by volume, respectively. The cells displayed a spindle shape, similar to that of the positive control cells. **D)** Negative control cells, characterized by their rounded shape. Scale bars: 100 $\mu$m.

**Figure 2.** WST-1 cell proliferation assay results. The values are shown expressed as the mean $\pm$ standard deviation of 6 independent experiments for each experimental group. *p=0.000, compared with the other experimental groups.

**Figure 3.** Free DNA quantification results. The values are shown expressed as the mean $\pm$ standard deviation from the mean. Six independent experiments were performed for each experimental group. *p < 0.05, compared with the other experimental groups.

**Figure 4.** Light microscopy of the sample. **A)** Control sample (non-magnetic) gelled in the absence of an applied magnetic field (Ctrl-MFO). **B)** Magnetic sample gelled in the absence of an applied magnetic field (Ctrl-MFO). **C)** Magnetic sample gelled under the application of 32 kA/m (M-MF32). The arrows indicate the presence of some cells in the biomaterial.

**Figure 5.** Scanning microscopy of the samples. A) Control sample (non-magnetic) gelled in the absence of an applied magnetic field (Ctrl-MFO). **B)** Magnetic sample gelled in the absence of an applied magnetic field (M-MF0). **C)** Magnetic sample gelled under the application of a magnetic field of 48 kA/m (M-MF48).

**Figure 6.** Live & dead viability assays - percentage of viable cells. The mean $\pm$ standard deviation values of 6 independent experiments for each experimental group are shown.

**Figure 7.** Biomaterial DNA quantification results. The mean $\pm$ standard deviation values of 6 independent experiments are shown for each experimental group.

**Figure 8.** Magnetization as a function of the strength of the magnetic field applied for different magnetic tissue samples. Solid squares: M-MFO; hollow circles: M-MF16; hollow triangles: M-MF32; solid triangles: M-MF48.

**Figure 9. A)** Modulus of elasticity as a function of the shear strain amplitude in oscillometry testing at the frequency of 1 Hz. **B)** Shear stress as a function of strain obtained in steady-state tests. Measurements performed in the absence of magnetic field.

**Figure 10.** Normalized modulus of elasticity, $\dfrac{G' - G'_{control}}{\phi_{MagNP-OH} \cdot G'_{control}}$, as a function of the shear strain amplitude for the magnetic tissue samples that are indicated.

**Figure 11. A)** Modulus of elasticity as a function of the shear strain amplitude for sample M-MF32 under the action of the magnetic fields the strength of which, H, is indicated. **B)** Shear stress vs. strain for sample M-MF16 obtained in steady-state tests under the application of the magnetic fields the strength of which is indicated.

## DETAILED DESCRIPTION OF THE INVENTION

The present invention relates to a biomaterial comprising multiple magnetic domain particles having a mean size greater than 25 nm, preferably about 100 nm, for use in tissue regeneration. Therefore, the present invention provides an *in vitro* method for producing an artificial tissue, the artificial tissue that can be produced by said method, and the use of said artificial tissue for partially or completely increasing, restoring or replacing the functional activity of a damaged organ or tissue. The introduction of magnetic particles in artificial tissues allows increasing their mechanical strength, and it further allows controlling their biomechanical properties in a non-invasive manner by means of remotely acting magnetic forces that do not require direct contact with the generated tissue. This makes magnetic tissues more versatile and more adaptable compared with non-magnetic tissues. Furthermore, the magnetic tissues could be fixed by means of magnetic forces exerted from outside the tissue. Surgical sutures and their adverse effects could therefore be avoided or minimized.

*PARTICLES AND COMPOSITIONS OF THE INVENTION*

The multiple magnetic domain particle has a mean diameter greater than 25 nm, hereinafter *"magnetic particle of the invention."* More preferably, the mean diameter of the particles is between 25 and 5000 nm, even more preferably it is between 50 and 1000 nm, even much more preferably between 75 and 200 nm, and particularly about 100 nm.

In another preferred embodiment, the magnetic particles of the invention are of a ferro- or ferrimagnetic material, preferably iron or alloys thereof with other metals, and even more preferably iron oxides. In a preferred embodiment, the particles will be of maghemite or a ferrite, and among the latter preferably of magnetite.

In another preferred embodiment, the magnetic particles of the invention are coated with a polymeric material that is preferably biocompatible and/or biodegradable. The polymeric material is selected from polylactic acid, polyglycolic acid, chitosan, poly($\epsilon$-caprolactone), PLGA, a mis-methacrylate polymer, poly(alkyl cyanoacrylate), polyethyleneimine (PEI), poly(L-lysine) (PLL), poly(2-dimethyl-amino)ethylmethacrylate (pDMAEMA), polypeptides derived from histidine, poly(D,L-lactide) (PLA), poly(glycolide) (PGA), PLGA, poly($\epsilon$-caprolactone), PEG, poly (N-(2-hydroxypropyl)methacrylamide) (PHPMA), or a derivative or salt thereof, or any of the mixtures thereof. More preferably it is poly(alkyl cyanoacrylate), or a derivative or salt thereof, or any of the mixtures thereof. In another more preferred embodiment, the polymeric material is selected from a methacrylate polymer, polyethyleneimine (PEI), poly(L-lysine) (PLL), poly(2-dimethyl-amino)ethylmethacrylate (pDMAEMA), polypeptides derived from histidine, poly(D,L-lactide) (PLA), poly(glycolide) (PGA), PLGA, poly($\epsilon$-caprolactone), PEG, poly (N-(2-hydroxypropyl)methacrylamide) (PHPMA), or any combination thereof.

In the scope of the present invention, the term "*salts*" includes, without limitation, e.g. nitrate, phosphate, sulfate, hydrochloride, glutamate, lactate or acetate. The term derivative includes, without limitation, esters, ethers, derivatives from the bonding of acyl groups, alkyl groups, hydroxyl groups, etc). In another preferred embodiment of the invention, the derivatives are selected from alkyl ether methacrylates and acyl esters of methacrylates.

In another more preferred embodiment, particles consist of a magnetite core ($\gamma$-Fe$_3$O$_4$) and are coated with a polymer matrix of methyl methacrylate-co.hydroxylethyl methacrylate-co-ethylene glycol dimethacrylate (MMA-co-HEMA-co-EGDMA).

The *"mean size"* or *"mean diameter"* of these systems can be measured by means of standard methods known by those skilled in the art and are described, for example, in the experimental part below.

*"Mean diameter"* is understood as the average diameter of the population of particles dispersed in an aqueous medium. The mean diameter of these systems can be measured by standard methods known by those skilled in the art and are described, for example, in the examples below.

*ARTIFICIAL TISSUE OF THE INVENTION*

The invention relates to an artificial tissue, hereinafter *"artificial tissue of the invention,"* as defined in claim 1.

The magnetic particles that are added in step (c), in the presence of the magnetic field, form columns, and reinforce the structure. In other words, a magnetic field is applied after step (e) to form the structures and additionally reinforce tissue elasticity.

Preferably, the applied magnetic field is less than 500 kA/m, more preferably less than 100 kA/m, and even more preferably it is comprised between 10 and 50 kA/m. Even much more preferably, it is comprised between 16 and 48 kA/m.

Said magnetic field will preferably be generated in the region where the tissue containing magnetic particles is located and can be produced by means of coils through which a direct electric current circulates. It will preferably be a magnetic field that is uniform in space in the tissue containing magnetic particles

In step (a) of the method of the invention, a composition comprising fibrinogen is added to isolated cells, preferably cells isolated from a mammal. Said cells can be obtained by means of different methods described in the state of the art and they can depend on the particular cell type in question. Some of these methods are, for example, without limitation, biopsy, mechanical processing, enzyme treatment (for example, without limitation, with trypsin or collagenase type I), centrifugation, white blood cell lysis, filtration, culturing in supports or media favoring the selective proliferation of said cell type or immunocytometry. Some of these methods are described in detail in the examples of this specification.

The cells of step (a) can be differentiated cells such as, without limitation, fibroblasts, keratocytes or smooth muscle cells, or undifferentiated cells with the capacity to differentiate into said cells, such as adult stem cells, for example.

In a preferred embodiment of the method of the invention, the cells of step (a) are fibroblasts or undifferentiated cells with the capacity to differentiate into fibroblasts. The fibroblasts can be obtained from any tissue or organ; however, the fibroblasts of step (a) are preferably from the tissue or organ in which the artificial tissue is to be used as a replacement.

The possibility that all the components of the artificial tissue are autologous allows being able to transplant said tissue without requiring immunosuppression of the transplanted subject. However, the components of the artificial tissue can also be allogeneic, i.e., they can come from an individual other than the individual in whom the artificial tissue is going to be transplanted. Even the species which said components come from can be different; in this case, these components are said to be xenogeneic. This opens up the possibility of the artificial tissue being produced beforehand when it is urgently needed, although in this case it would be recommendable to immunosuppress the subject in whom the artificial tissue is transplanted.

Therefore, in a preferred embodiment, the cells of step (a) of the invention are autologous. Nevertheless, the cells of step (a) can also be allogeneic or xenogeneic.

By means of adding the different components described in steps (a)-(d) of the method of the invention to the cells of step (a), and after allowing the product resulting from step (e) to settle in a support, a matrix comprising fibrin, the polysaccharide, and where appropriate, the protein added in step (d2) if said step was applied, is formed, in which said cells are embedded and on which and/or inside of which said cells can grow. Preferably, the cells of step (a) grow in the inner of said matrix.

The formation of a fibrin matrix takes place through thrombin-induced fibrinogen polymerization. Fibrinogen is a high molecular weight protein present in blood plasma. Thrombin is a proteolytic enzyme that causes the fibrinogen molecule to break up into low molecular weight polypeptides and fibrin monomers. Said monomers polymerize into dimers and then bind to one another by means of covalent bonds through the action of factor XIII previously activated by thrombin and in the presence of calcium ions.

The composition comprising fibrinogen of step (a) can be, for example, without limitation, blood plasma. The composition of step (a) can also be produced from a plasma derivative, such as, without limitation a cryoprecipitate or a fibrinogen concentrate. In addition to fibrinogen, the composition of step (a) can contain other coagulation factors.

In a preferred embodiment, the fibrinogen concentration in the product resulting from step (c) is between 0.5 and 10 g/L, optionally between 1 and 10 g/L. In a more preferred embodiment, the concentration in the product resulting from step (e) is between 1 and 4 g/L, optionally between 2 and 4 g/L. Nevertheless, a higher or lower concentration could also be used.

In a preferred embodiment, the fibrinogen of the composition of step (a) or the composition comprising fibrinogen of step (a) is autologous. Nevertheless, the fibrinogen of the composition of step (a) or the composition comprising fibrinogen of step (a) can also be allogeneic or xenogeneic.

In a preferred embodiment of this first aspect of the invention, the fibrinogen-containing composition of step (a) is blood plasma. In this case, fibrinogen polymerization can be induced by means of adding a source of calcium in step (c).

In a more preferred embodiment of this first aspect of the invention, the source of calcium of step (c) is a calcium salt such as, without limitation, calcium chloride, calcium gluconate or a combination of both. The calcium salt concentration must be enough to induce fibrinogen polymerization. In a more preferred embodiment, the calcium salt is calcium chloride. In an even more preferred embodiment, the calcium chloride concentration in the product resulting from step (f) is between 0.25 and 3 g/L, optionally between 0.5 and 4 g/L. Nevertheless, a higher or lower concentration could also be used.

As it is used in the present description, the term *"coagulation factor"* refers to a component, generally a protein, present in blood plasma and involved in the chain reaction that enables coagulation. There are thirteen coagulation factors, named using Roman numerals: I: fibrinogen; II: prothrombin; III: tissue factor or thromboplastin; IV: calcium; V: proaccelerin; VI: inactive factor or zymogen; VII: proconvertin; VIII: antihemophilic factor A or von Willebrand factor; IX: antihemophilic factor B or Christmas factor; X: Stuart-Prower factor; XI: antihemophilic factor C; XII: Hageman factor; XIII: fibrin stabilizing factor; XIV: Fitzgerald; XV: Fletcher; XVI: platelets; and XVII: Somocurcio. Preferably, the other coagulation factor added in step (c) of the method of the present invention is factor XIII.

Fibrin polymer can be degraded by means of the process called fibrinolysis. During fibrinolysis, plasminogen is converted into active plasmin enzyme by the plasminogen tissue activator; the plasmin binds to the fibrin surface through its binding sites to cause fibrin polymer degradation. To prevent fibrinolysis of the fibrin matrix, an antifibrinolytic agent is added in step (b) of the present invention such as, without limitation, epsilon aminocaproic acid, tranexamic acid or aprotinin.

Tranexamic acid is a synthetic product derived from the amino acid lysine with high affinity for the lysine binding sites of plasminogen; it blocks these sites and prevents the binding of activated plasminogen to the fibrin surface, exerting an antifibrinolytic effect. Tranexamic acid has the advantage, in comparison with other antifibrinolytic agents of animal origin, that it does not transmit diseases. Therefore, in a preferred embodiment, the antifibrinolytic agent is tranexamic acid. In an even more preferred embodiment, the concentration of tranexamic acid in the product resulting from step (e) is between 0.5 and 2 g/L, preferably between 1 and 2 g/L. Nevertheless, a higher or lower concentration could also be used.

Fibrin matrices are very versatile, therefore they have been used for producing different artificial tissues, however, the clinical use thereof has been limited due mainly due to their limited consistency, their difficult manipulation and their extreme fragility. For that reason, a polysaccharide is added in step (e) of the method of the invention. Said polysaccharide is generally used to provide resistance and consistency to the tissue, and it is convenient that the polysaccharide is soluble therein. Examples of polysaccharides which can be used in step (e) of the method of the present invention are, without limitation, agar-agar, agarose, alginate, chitosan or carrageenans, or any combination of the above.

Agarose is a polysaccharide formed by alpha and beta galactoses extracted from algae of the genera such as *Gellidium* or *Gracillaria.* Agarose, in comparison with other polysaccharides which can be used in step (e) of the present invention, has the advantage that it forms a matrix which is inert from the immunological view point. Therefore, in a preferred embodiment, the polysaccharide of step (e) of the method of the invention is agarose. There are different types of agarose which vary in their physical and chemical properties such as, for example, gelling temperature, gel strength and/or porosity. Preferably, the agarose of step (e) of the method of the invention is an agarose with a low melting point, i.e., an agarose which repolymerizes and solidifies at a temperature, preferably, less than 65°C and, more preferably, less than 40°C; it can thus be used for producing the tissue at very low temperatures, minimizing the probability of cell death. In a more preferred embodiment, the agarose used in step (e) of the method of the invention is agarose type VII. In an even more preferred embodiment, the agarose, preferably, agarose type VII, in the product resulting from step (e) is at a concentration, advantageously between 0.1 and 6 g/L, optionally between 0.2 and 6 g/L, preferably between 0.15 and 3 g/L, optionally between 0.3 and 3 g/L and more preferably between 0.25 and 2 g/L, optionally between 0.5 and 2 g/L. Nevertheless, a higher or lower concentration could also be used.

In a preferred embodiment, the method of the invention comprises an additional step between step (b) and step (c) (step (b2)) in which a protein is added. Examples of proteins that can be used in step (b2) of the method of the present invention are, without limitation, fibronectin, laminin, collagen type VII or entactin, or any combination of the above. These proteins naturally form part of the extracellular matrix of the connective tissue in tissues, therefore the cells embedded in an artificial tissue produced by means of the method of the invention at a microenvironment which is more similar to a physiological environment, improving the adhesion, differentiation and/or survival of said cells.

In a preferred embodiment, the protein that is added in step (b2) is fibronectin. Fibronectin is a glycoprotein present in the extracellular matrix (ECM) of most animal cellular tissues playing an important role in matrix cell adhesion. In a more preferred embodiment, the protein added between step (b) and step (c) of the method of the invention is fibronectin. The object of this addition is to favor adhesion of the cells of step (f) to the product resulting from step (e). For example, when the method of the invention is used for producing a cornea replacement tissue or an artificial cornea, adding fibronectin reduces the detachment of corneal epithelial cells added in step (f) which involves a significant advantage with respect to other methods described in the prior art. In an even more preferred embodiment, the fibronectin concentration in the product resulting from step (e) is between 0.25 and 1 g/L, optionally between 0.5 and 1 g/L. Nevertheless, a higher or lower concentration could also be used.

In a preferred embodiment, the method of the invention comprises an additional step (step e2) between steps (e) and (f) which comprises adding a composition comprising a protein to the product resulting from step (e). Examples of proteins that can be used in step (f) of the method of the present invention are, without limitation, collagen, reticulin or elastin. Adding a protein between step (e) and step (f) yields tissues having a greater fibril density at the stroma level, better viscoelastic behavior and an increasing threshold stress. In an even more preferred embodiment, the protein that is added in step (e2) is collagen.

Adding said proteins in step (e2) of the method of the invention also improves the physical (rheological, mechanical or biomechanical) properties of the artificial tissue produced. The examples of this specification demonstrate that using increasing collagen concentrations in artificial tissues comprising fibrin, agarose and collagen improves the viscoelastic behavior, which is clearly shown by an increase in the collagen concentration-dependent threshold stress.

The main rheological properties of a solid or semisolid material are the viscosity and elasticity. Viscosity is the resistance of a fluid against tangential strain, and it would be equivalent to consistency or rigidity. Elasticity is the mechanical property of certain materials of undergoing reversible deformations when they are subjected to the action of external forces, and to recover the original shape when these external forces cease. These parameters are analyzed by means of rheometry, a physical technique using instruments called rheometers.

Threshold stress is the mechanical force needed to cause an irreversible deformation in a solid or fluid. Normally, all materials have an elastic region, in which the force applied causes a completely reversible deformation when the force ceases. If that force exceeds a limit (modulus of elasticity), the deformation becomes irreversible, entering into a plastic region. Finally, if the force exceeds the plastic modulus, the material breaks (yield point).

Collagen is a protein which is readily available in nature and is biologically characterized by its low immunity and high tissue activity. Collagen forms collagen fibers, which are flexible but offer great tensile strength. The artificial tissues of the present invention have a greater fibril density at the stroma level, better viscoelastic behavior and an increasing threshold stress as the collagen concentration increases, and higher than artificial collagen tissues. Therefore, in a preferred embodiment the protein added in step (f) is collagen.

In a preferred embodiment, the collagen added in step (e2) is selected from the list comprising: collagen type I, collagen type II, collagen type III, collagen type IV, collagen type V, collagen type VI, collagen type VII, collagen type VIII, collagen type IX, collagen type X, collagen type XI, collagen type XII, collagen type XIII or any combination of the above. In a more preferred embodiment, the collagen added in step (f) is selected from the list comprising: collagen type I, collagen type II, collagen type III, collagen type IV, collagen type V, collagen type IX or any combination of the above. The selection of a particular type of collagen in step (f) of the method of the invention depends on the artificial tissue to be produced and is performed according to the characteristics of each collagen that are known in the state of the art.

For example, the main function of collagen type I is to resist against stretching, and it is found abundantly in the dermis, bone, tendon and cornea. Therefore, the present invention demonstrates that adding collagen type I in step (f) renders excellent properties to the artificial tissue when, for example, without limitation, a cornea replacement tissue or an artificial cornea is to be produced. Therefore, in a preferred embodiment the collagen is collagen type I.

In an even more preferred embodiment, the collagen, preferably, collagen type I, in the product resulting from step (e) is at a concentration advantageously between 0.5 and 5 g/L, preferably, between 1.8 and 3.7 g/L, and more preferably, between 2.5 and 3 g/L. Nevertheless, a higher or lower concentration could also be used.

In a particular embodiment, the collagen used is an atelocollagen, i.e., a collagen from which the terminal regions of non-helical structure called telopeptides have been removed. These telopeptides are the carriers of the main antigenic determinants of the collagen and can make the collagen insoluble. Atelocollagen is obtained, for example, by means of protease treatment with pepsin.

Depending on the fibrinogen concentrations used in step (a), on the polysaccharide concentration used in step (e), and in the event that a step (e2) is applied, on the collagen concentration used in step (e2), the artificial tissue resulting from step (f) can comprise variable concentrations of two/three components.

In a preferred embodiment, the fibrinogen concentration in the product resulting from step (f) is between 0.5 and 10 g/L, the concentration of the agarose, preferably, agarose type VII, is between 0.1 and 6 g/L. If step (e2) has been included, the concentration of the collagen, preferably, collagen type I, is between 0.5 and 5 g/L.

In another preferred embodiment, the fibrinogen concentration in the product resulting from step (f) is between 0.5 and 10 g/L, the concentration of the agarose, preferably, agarose type VII, is between 0.15 and 3 g/L. If step (e2) has been included, the concentration of the collagen, preferably, collagen type I, is between 0.5 and 5 g/L.

In another preferred embodiment, the fibrinogen concentration in the product resulting from step (f) is between 0.5 and 10 g/L, the concentration of the agarose, preferably, agarose type VII, is between 0.25 and 2 g/L. If step (e2) has been included, the concentration of the collagen, preferably, collagen type I, is between 0.5 and 5 g/L.

In another preferred embodiment, in the product resulting from step (e), the fibrinogen concentration is between 0.5 and 10 g/L, the concentration of the agarose, preferably, agarose type VII, is between 0.1 and 6 g/L. If step (e2) has been included, the concentration of the collagen, preferably, collagen type I, is between 1.8 and 3.7 g/L.

In another preferred embodiment, the fibrinogen concentration in the product resulting from step (f) is between 0.5 and 10 g/L, the concentration of the agarose, preferably, agarose type VII, is between 0.15 and 3 g/L. If step (e2) has been included, the concentration of the collagen, preferably, collagen type I, is between 1.8 and 3.7 g/L.

In another preferred embodiment, the fibrinogen concentration in the product resulting from step (f) is between 0.5 and 10 g/L, the concentration of the agarose, preferably, agarose type VII, is between 0.25 and 2 g/L. If step (e2) has been included, the concentration of the collagen, preferably, collagen type I, is between 1.8 and 3.7 g/L.

In another preferred embodiment, the fibrinogen concentration in the product resulting from step (f) is between 0.5 and 10 g/L, the concentration of the agarose, preferably, agarose type VII, is between 0.1 and 6 g/L. If step (e2) has been included, the concentration of the collagen, preferably, collagen type I, is between 2.5 and 3 g/L.

In another preferred embodiment, the fibrinogen concentration in the product resulting from step (f) is between 0.5 and 10 g/L, the concentration of the agarose, preferably, agarose type VII, is between 0.15 and 3 g/L. If step (e2) has been included, the concentration of the collagen, preferably, collagen type I, is between 2.5 and 3 g/L.

In another preferred embodiment, the fibrinogen concentration in the product resulting from step (f) is between 0.5 and 10 g/L, the concentration of the agarose, preferably, agarose type VII, is between 0.25 and 2 g/L. If step (e2) has been included, the concentration of the collagen, preferably, collagen type I, is between 2.5 and 3 g/L.

In another preferred embodiment, the fibrinogen concentration in the product resulting from step (f) is between 1 and 4 g/L, the concentration of the agarose, preferably, agarose type VII, is between 0.1 and 6 g/L. If step (e2) has been included, the concentration of the collagen, preferably, collagen type I, is between 0.5 and 5 g/L.

In another preferred embodiment, the fibrinogen concentration in the product resulting from step (f) is between 1 and 4 g/L, the concentration of the agarose, preferably, agarose type VII, is between 0.15 and 3 g/L. If step (e2) has been included, the concentration of the collagen, preferably, collagen type I, is between 0.5 and 5 g/L.

In another preferred embodiment, the fibrinogen concentration in the product resulting from step (f) is between 1 and 4 g/L, the concentration of the agarose, preferably, agarose type VII, is between 0.25 and 2 g/L. If step (e2) has been included, the concentration of the collagen, preferably, collagen type I, is between 0.5 and 5 g/L.

In another preferred embodiment, the fibrinogen concentration in the product resulting from step (f) is between 1 and 4 g/L, the concentration of the agarose, preferably, agarose type VII, is between 0.1 and 6 g/L. If step (e2) has been included, the concentration of the collagen, preferably, collagen type I, is between 1.8 and 3.7 g/L.

In another preferred embodiment, the fibrinogen concentration in the product resulting from step (f) is between 1 and 4 g/L, the concentration of the agarose, preferably, agarose type VII, is between 0.15 and 3 g/L. If step (e2) has been included, the concentration of the collagen, preferably, collagen type I, is between 1.8 and 3.7 g/L.

In another preferred embodiment, the fibrinogen concentration in the product resulting from step (f) is between 1 and 4 g/L, the concentration of the agarose, preferably, agarose type VII, is between 0.25 and 2 g/L. If step (e2) has been included, the concentration of the collagen, preferably, collagen type I, is between 1.8 and 3.7 g/L.

In another preferred embodiment, the fibrinogen concentration in the product resulting from step (f) is between 1 and 4 g/L, the concentration of the agarose, preferably, agarose type VII, is between 0.1 and 6 g/L. If step (e2) has been included, the concentration of the collagen, preferably, collagen type I, is between 2.5 and 3 g/L.

In another preferred embodiment, the fibrinogen concentration in the product resulting from step (f) is between 1 and 4 g/L, the concentration of the agarose, preferably, agarose type VII, is between 0.15 and 3 g/L. If step (e2) has been included, the concentration of the collagen, preferably, collagen type I, is between 2.5 and 3 g/L.

In another preferred embodiment, the fibrinogen concentration in the product resulting from step (f) is between 1 and 4 g/L, the concentration of the agarose, preferably, agarose type VII, is between 0.25 and 2 g/L. If step (e2) has been included, the concentration of the collagen, preferably, collagen type I, is between 2.5 and 3 g/L.

By means of adding the different components described in steps (a)-(e) of the method of the invention to the cells of step (a), and after allowing the product resulting from step (f) to settle in a support, a matrix comprising fibrin, the particles of the invention, the polysaccharide, and in the event that step (e2) has been included, the protein added in said step, is formed, in which said cells are embedded and on which and/or inside of which said cells can grow. Preferably, the cells of step (a) grow in the inner of said matrix.

Once steps (a)-(e) of the method of the invention are carried out, the product resulting from step (e) is left to settle in a support to cause formation of the matrix comprising the fibrin, the polysaccharide, and depending on if step (b2) has been carried out, the protein added in said step, and having the cells of step (a) embedded therein. Supports which can be used are, for example, without limitation, tissue culture plates or porous cell culture inserts. Preferably, said supports will be in sterile conditions.

Step (f) of the method of the invention consists of culturing isolated cells, preferably cells isolated from a mammal, in or on the product resulting from step (f). Said cells can be obtained by means of different methods described in the state of the art and they can depend on the particular cell type in question. Some of these methods are, for example, without limitation, biopsy, mechanical processing, enzyme treatment (for example, without limitation, with trypsin or collagenase type I), centrifugation, white blood cell lysis, filtration, culturing in supports or media favoring the selective proliferation of said cell type or immunocytometry. Some of these methods are described in detail in the examples of this invention.

The cells of step (f) can be differentiated cells such as, without limitation, epithelial cells, or undifferentiated cells with the capacity to differentiate into said cells, such as adult stem cells, for example.

In a preferred embodiment, the differentiated cells of step (f) are epithelial cells, such as, without limitation, keratinocytes, epithelial cells of the oral mucosa, epithelial cells of the bladder, epithelial cells of the urethra, corneal epithelial cells or vascular endothelial cells.

Preferably, the epithelial cells of step (f) are from the tissue or organ in which the artificial tissue is to be used as a replacement. For example, when the method of the invention is used to produce a skin replacement tissue or an artificial skin, the epithelial cells are preferably from the epidermis of the skin, i.e., they are keratinocytes; when they are used to produce a bladder replacement tissue or artificial bladder, the epithelial cells are preferably from the epithelium of the bladder or urothelium; when used to produce a urethra replacement tissue or an artificial urethra the epithelial cells are preferably from the epithelium of the urethra; when used to produce a cornea replacement tissue or an artificial cornea the epithelial cells are preferably corneal epithelial cells; when used to produce an oral mucosa replacement tissue, the epithelial cells are preferably from the epithelium of the oral mucosa.

However, the epithelial cells of step (f) can also be obtained from a tissue or organ other than the tissue or organ in which the artificial tissue is to be used as a replacement. For example, when the method of the invention is used to produce a skin replacement tissue or an artificial skin, a bladder replacement tissue or an artificial bladder, a urethra replacement tissue or an artificial urethra, or a cornea replacement tissue or an artificial cornea, the epithelial cells of step (f) can be epithelial cells of the oral mucosa., or for example, when the method of the invention is used to produce a bladder replacement tissue or an artificial bladder, or when used to produce a urethra replacement tissue or an artificial urethra, the epithelial cells can be keratinocytes.

One of the problems associated with artificial tissue generation in a laboratory is the production of a significant number of differentiated cells, so the use of stem cells with the capacity to differentiate into said cells is often considered as an alternative source. "Stem cell" is understood as that having a high capacity for dividing and morphologically and functionally differentiating into different types of more specialized cells. During the differentiation process, an undifferentiated cell changes its phenotype and morphology to be converted into a differentiated cell with a specialized structure and function.

Stem cells can be classified, according to their potential, i.e., their capacity for differentiating into different cell types, as: (a) totipotentials (not claimed): capable of differentiating both into embryonic tissue and extraembryonic tissue; (b) pluripotentials with the capacity to differentiate into any of the tissues originating from the three embryonic layers (endoderm, mesoderm and ectoderm); (c) multipotentials: capable of differentiating into different cell types derived from one and the same embryonic layer (endoderm, mesoderm or ectoderm); and (d) unipotentials: capacity to form a single cell lineage.

According to their origin, the stem cells have been divided into: (a) embryonic stem cells: from the inner cell mass of the blastula in the preimplantation stage or gonadal crest stage, which are totipotentials (not claimed) or pluripotentials; and (b) adult stem cells: in the adult, the fetus and the umbilical cord, which are multipotentials or unipotentials. Mesenchymal stem cells which are distributed in the connective tissue of different organs, such as, without limitation, bone marrow, peripheral blood, adipose tissue or umbilical cord, are included among adult stem cells. In a preferred embodiment, the adult stem cells are adult stem cells from the bone marrow, adipose tissue or umbilical cord.

The umbilical cord is an interesting source of adult stem cells due to the fact that, unlike the adult stem cells obtained from other sources: (a) the method for obtaining them is not invasive or painful; and (b) their proliferative capacity and differentiation potential do not drop as a result of aging. Among the different sources of umbilical cord stem cells, the so-called umbilical cord Wharton's jelly stem cells stand out due to: (a) their great proliferation capacity and their speed of spreading in culture; and (b) the low expression of Class I major histocompatibility complex and the absence of expression of Class II major histocompatibility complex, making them good candidates for allogeneic cell therapy.

Therefore, in another preferred embodiment, the cells of step (f) are umbilical cord Wharton's jelly stem cells. These cells express different mesenchymal cell characteristic markers on their surface such as, for example, SH2, SH3, CD10, CD13, CD29, CD44, CD54, CD73, CD90, CD105 or CD166, and they are negative for hematopoietic lineage markers, such as, for example, CD31, CD34, CD38, CD40 or CD45. Umbilical cord Wharton's jelly stem cells can differentiate, for example, into chondroblasts, osteoblasts, adipocytes, neural precursors, cardiomyocytes, skeletal muscle cells, endothelial cells or hepatocytes.

Adult stem cells can be characterized by means of identifying surface and/or intracellular proteins, genes, and/or other markers indicative of their undifferentiated state, by means of different methods which are known in the state of the art such as, without limitation, immunocytometry, immunocytochemical analysis, northern blot analysis, RT-PCR, gene expression analysis in microarrays, proteomic studies or differential display analysis.

Stem cells can be induced to differentiate *in vitro* to produce cells expressing at least one or more typical characteristics of differentiated cells. Examples of differentiated cells which can be differentiated from the stem cells are, without limitation, fibroblasts, keratinocytes, urothelial cells, epithelial cells of the urethra, corneal epithelial cells, epithelial cells of the oral mucosa, chondroblasts, osteoblasts, adipocytes or neurons. In a preferred embodiment of the invention, the cell differentiated from the multipotent stem cell of the invention expresses one or more typical characteristics of a differentiated cell selected from the list comprising: fibroblasts, keratinocytes, urothelial cell, epithelial cell of the urethra, corneal epithelial cell, epithelial cell of the oral mucosa, chondroblast, osteoblast, adipocyte or neuron.

The differentiated cells can be characterized by means of identifying the surface and/or intracellular proteins, genes, and/or other markers indicative of their differentiated state, by means of different methods which are known in the state of the art such as, without limitation, immunocytometry, immunocytochemical analysis, northern blot analysis, RT-PCR, gene expression analysis in microarrays, proteomic studies or differential display analysis.

In a preferred embodiment, the cells of step (f) of the invention are autologous. Nevertheless, the cells of step (f) can also be allogeneic or xenogeneic.

The cells of step (f) are capable of proliferating on the product resulting from step (e) and/or inside said product. Preferably, the cells of step (f) proliferate on the surface of the product resulting from step (e).

The cells of step (f) are left to proliferate until reaching a suitable number, typically at least, 70% of confluence, advantageously, at least, 80% of confluence, preferably, at least, 90% of confluence, more preferably, at least, 95% of confluence, and even more preferably, at least, 100% of confluence. During the time the cells are kept in culture, the culture medium in which they are located can be partially or completely replaced with fresh medium in order to replace depleted ingredients and remove potentially harmful metabolites and catabolites.

An additional step may be necessary for the correct differentiation of some cell types. For example, in the case of epithelial cells of the oral mucosa, keratinocytes or corneal epithelial cells, it may be necessary to expose the epithelial surface to air to encourage correct epithelium stratification and maturation by keeping the matrix comprising the cells of step (a) submerged in culture medium (air-liquid technique).

Therefore, in a preferred embodiment, the method of the invention, in addition to steps (a)-(g) described above, comprises an additional step in which the product resulting from step (f) is exposed to air. The method of the invention generally includes this step when it is used to produce an artificial tissue for replacing a natural tissue the epithelium of which is usually exposed to contact with air such as, without limitation, the skin, the cornea, the oral mucosa, the urethra or the vagina. Preferably, this step is performed when a skin replacement tissue or an artificial skin is produced, or when a cornea replacement tissue or an artificial cornea is produced, or when an oral mucosa replacement tissue or an artificial oral mucosa is produced.

One of the most significant innovations of the method of the invention consists of the existence of a step (g) in which the nanostructuring of the product resulting from step (f) is induced. As used herein, the expression "nanostructuring" refers to a structural modification consisting of generating bonds having a size of less than one micron between the fibrin fibers and between fibrin fibers and agarose molecules. This nanostructuring process allows obtaining artificial tissues which unexpectedly show advantageous properties with respect to non-structured biomaterials. Specifically, the biomaterials subjected to a nanostructuring process according to the present invention have (i) a significant improvement of tissue biomechanical properties, which allows manipulating the nanostructured tissue and it involves a substantial and unexpected improvement of the biomaterial rheological properties, characterized as a greater resistance and a greater elasticity; (ii) a substantial improvement of the nanostructured tissue manipulability, which allowed its surgical manipulation, suturing to the recipient bed and implanting in test animals, (iii) a significant improvement of the transparency of the tissue subjected to nanostructuring, which is not completely predictable since the nanostructured tissues are denser and have a lower water content than the non-nanostructured tissues; and (iv) a better clinical result once implanted in laboratory animals which, on one hand, is related to the greater clinical implant efficiency due to the suitable biomechanical properties of the biomaterial and, on the other hand, to the fact that the biomaterials subjected to nanostructuring have a greater fiber density per $mm^2$, and therefore, slower remodeling by the receiving organism.

In a preferred embodiment, the induction of the nanostructuring of step (g) comprises the dehydration and/or mechanical compression of the product resulting from step (f). The objective of step (g) is to generate a structural modification between the fibrin fibers and the agarose molecules of the artificial tissue in order to reach optimal consistency and elasticity levels, which cannot be obtained by means of other methods described in the state of the art. The final result is an irreversible modification of the fibers, which generates biomechanical qualities that are very favorable for surgical manipulation and clinical implant.

The term *"dehydration"* refers to a partial and/or total removal of the interstitial fluid from the product resulting from step (f). For example, the amount of interstitial fluid removed from the product resulting from step (f) can be at least 50%, at least 60%, at least 70%, at least 80%, at least 90%, or at least 99% of the interstitial fluid originally contained in the product resulting from step (f).

The product resulting from step (f) can be dehydrated by means of any physical or chemical method. In a preferred embodiment, the dehydration of the product resulting from step (f) comprises a method selected from the list comprising: drainage, evaporation, suction, capillary pressure, osmosis or electro-osmosis.

The interstitial fluid can be removed by means of inclining the product resulting from step (f); the interstitial fluid is then drained due to gravity and gradient effect.

The fluid can be removed by means of suction, for example, by applying a vacuum to the surface where the product resulting from step (f) is located by means of a mechanical pump.

The interstitial fluid can be removed by means of evaporation, for example, by incubating the product resulting from step (f) in conditions that cause the evaporation, for example, at a pressure less than atmospheric pressure and/or at a temperature greater than room temperature.

The interstitial fluid can also be removed using an osmotic agent with water absorbing tendency such as, without limitation, a hyperosmotic sodium chloride solution, separating the product resulting from step (f) from this solution by means of a semi-permeable membrane, a sponge or another drying material.

In a preferred embodiment, the interstitial fluid can be removed by means of capillary pressure, for example, by means of applying an absorbent material to the product resulting from step (f). Some examples of absorbent material which could be used in step (g) of the invention, without limitation, are filter paper, Whatman 3M paper, cellulose fiber, or absorbent fabric. The absorbent material will preferably be sterilized.

The time required for dehydration will depend on the method or methods used, and it can be easily determined by a person skilled in the art. The suitability of the artificial tissue obtained by means of applying a specific dehydration method for a specific time period can be confirmed by means of different evaluation methods known in the state of the art such as, without limitation, those described in the examples of this specification.

The interstitial fluid can also be removed by means of the mechanical compression of the product resulting from step (f). The mechanical compression of the product resulting from step (f) can also impart a specific desired shape to the product resulting from step (f).

The product resulting from step (e) can be compressed by means of any method described in the state of the art. A "static" compression method can be used, wherein the product resulting from step (f) remains stationary such as, without limitation, the application of a static load (for example, a deadweight), a hydraulic element or a cam. A "dynamic" compression method can also be used, wherein the product resulting from step (f) moves during the compression such as, for example, by means of the application of one or more rollers or by means of extrusion through a constricting hole.

The product resulting from step (f) can be mechanically compressed by means of extrusion, for example, by means of passing the product resulting from step (f) through a hole constricting it, for example, a conical chamber. The conical chamber can have porous walls, such that it would allow the removal of interstitial fluid from the product resulting from step (f) while it passes through the same.

The product resulting from step (f) can be compressed by means of centrifuging the product resulting from step (f). For example, the product resulting from step (f) can be placed on a tube with the porous bottom, such that, in addition to the mechanical compression, the removal of interstitial fluid from the product resulting from step (f) would occur.

The product resulting from step (f) can be compressed by means of applying a balloon therein to compress the product resulting from step (f) against a solid surface. The solid surface can, for example, form a tube surrounding the product resulting from step (e) allowing the formation of an artificial tubular tissue.

In a preferred embodiment, the compression of the product resulting from step (f) comprises applying a weight on top of the product resulting from step (f), such that a mechanical action of pressure is exerted on the tissue. It is obvious that the greater the weight the lesser the time needed for obtaining an artificial tissue with the suitable characteristics will be. The weight used for compressing can have a flat surface or can be placed on a material having a flat surface, for example, plastic, ceramic, metal or wood.

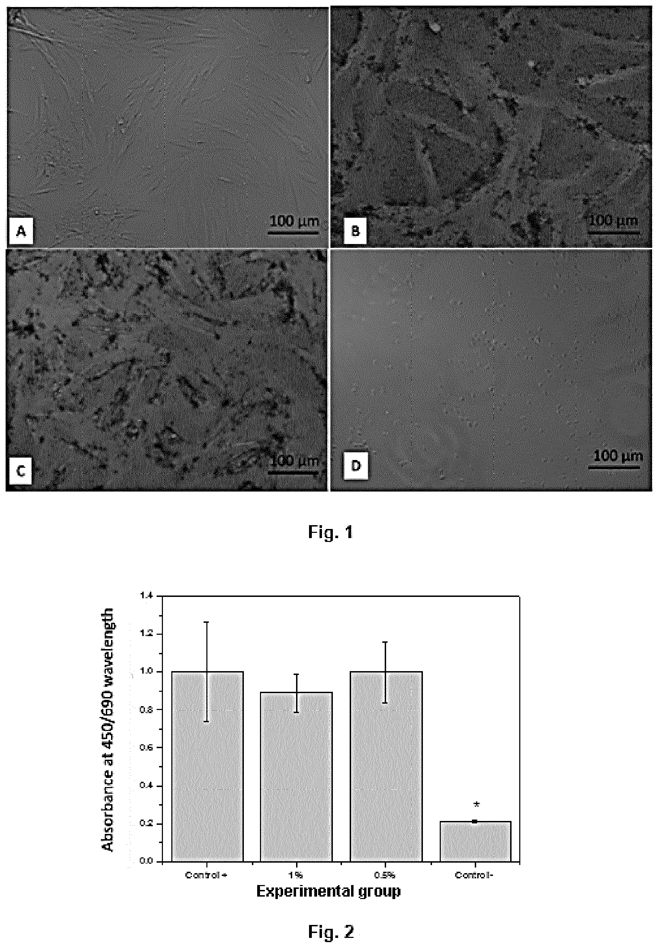

Figure 1 of the present specification shows a non-limiting diagram of how the nanostructuring of the product resulting from step (f) can be performed by means of the dehydration and compression thereof. It can be observed in said diagram how nanostructuring can be obtained by locating the product resulting from step (f) between two sterile filter papers, and placing thereon a weight of about 250 g (equivalent to about 2,000 N/m2) on a sterile flat glass surface for about 10 minutes; a porous material can be arranged between the tissue and the filter paper on which the weight is placed to prevent the product resulting from step (f) from adhering to the filter paper. The material used to prevent adherence must be porous to allow the exit of water from the tissue towards the dehydrating agent: said porous material used to prevent adherence can be, for example, without limitation, a nylon, glass, ceramic, perforated metal or polycarbonate membrane.

In a preferred embodiment, the compression of the product resulting from step (f) comprises applying a pressure thereon. The magnitude of the pressure is preferably between 1,000 and 5,000 $N/m^2$, more preferably between 1,500 and 2,500 $N/m^2$ and, even more preferably of about 2,000 $N/m^2$. Said pressure can be applied manually, automatically or semiautomatically. The time needed to exert the pressure depends on the magnitude of the pressure applied and it can be easily determined by a person skilled in the art. It is obvious that the greater the pressure the lesser the time needed for obtaining an artificial tissue with the suitable characteristics will be. The suitability of the artificial tissue obtained by means of applying a specific magnitude of pressure for a specific time period can be confirmed by means of different evaluation methods known in the state of the art such as, without limitation, those described in the examples of this specification.

One or more methods can be sequentially or simultaneously used to induce the nanostructuring of the product resulting from step (f). The time required for nanostructuring may be less than 12 hours, less than 6 hours, less than 3 hours, less than 1 hour, less than 30 minutes, less than 10 minutes, less than 2 minutes or less than 1 minute. The time required for nanostructuring will depend on the method or methods used, and it can be easily determined by the person skilled in the art. The suitability of the artificial tissue obtained by means of applying a specific method for a specific time period can be confirmed by means of different evaluation methods known in the state of the art such as, without limitation, those described in the examples of this specification.

Artificial tissue of the invention

A **first aspect** of the present invention relates to an artificial tissue that can be produced by the method of the invention described above (hereinafter, *"artificial tissue of the invention"*).

In a preferred embodiment of this first aspect of the invention, the artificial tissue of the invention is a skin replacement tissue or an artificial skin.

In another preferred embodiment of this first aspect of the invention, the artificial tissue of the invention is a bladder replacement tissue or an artificial bladder.

In another preferred embodiment of this first aspect of the invention, the artificial tissue of the invention is a urethra replacement tissue or an artificial urethra.

In another preferred embodiment of this first aspect of the invention, the artificial tissue of the invention is a cornea replacement tissue or an artificial cornea.

In another preferred embodiment of this first aspect of the invention, the artificial tissue of the invention is a mucosa replacement tissue or an artificial mucosa.

The artificial tissue that can be produced by the method of the invention can be cut to the desired size and/or can be provided in a suitable conformation for use.

Before use, the suitability of the artificial tissue of the invention for performing its function can be evaluated, for example, without limitation, by means of any of the methods described in the examples of the present description.

*USES OF THE ARTIFICIAL TISSUE OF THE INVENTION IN THE EVALUATION OF PHARMACOLOGICAL OR CHEMICAL PRODUCTS*

The medicinal products and chemical products must be evaluated in test animals before being marketed. In this regard, there are several reports and directives approved by the European Union which aim to restrict or even prohibit animal testing in the cosmetic product sector (Directive 76/768/EEC of the European Council relating to the approximation of Member States' laws on cosmetic products), and a complete ban is expected to be put into effect in the next few years. The European Union supports all the measures the main objective of which is the wellbeing of animals used for testing purposes and for achieving scientific replacement methods to reduce the number of animals used for testing to the minimum (Council Decision 1999/575/EEC of 23 March 1998, concerning the conclusion by the Community of the European Convention for the protection of vertebrate animals used for experimental and other scientific purposes - Official Journal L 222 of 24.08.1999).

Therefore, a **second aspect** of the invention relates to the use of the artificial tissue of the invention for the evaluation of a pharmacological and/or chemical product.

*THERAPEUTIC USES OF THE ARTIFICIAL TISSUES OF THE INVENTION*

An infectious, inflammatory, genetic or degenerative disease, physical or chemical damage, or blood flow interruption, can cause cell loss from a tissue or organ. This cell loss would lead to an alteration in the normal function of said tissue or organ, and consequently lead to the development of diseases or physical consequences reducing the person's quality of life. Therefore, attempting to regenerate and/or restore the normal function of said tissues or organs is important. The damaged tissue or organ can be replaced with a new tissue or organ which has been produced in the laboratory by means of tissue engineering techniques. The objective of tissue engineering is to construct artificial biological tissues and use them for medical purposes for restoring, replacing or increasing the functional activities of diseased tissues and organs. The therapeutic use of techniques of this type is virtually unlimited with applications in all fields. The use of tissue engineering techniques allows reducing the waiting lists for tissues and organs, with the consequent reduction of disease morbidity-mortality in the recipient. As a consequence, it also logically reduces the morbidity-mortality in organ donors. In addition, there are many advantages associated with the use of autologous cells or tissues in tissue engineering, which include: (a) a significant reduction of the number of donor to recipient infections by infectious agents;

and (b) the absence of host immune graft rejection, therefore the patient does not need to undergo immunosuppressing treatment, side effects and problems associated with immunodepression being prevented.

Therefore, a **third aspect** of the disclosure relates to the use of the artificial tissue of the invention for partially or completely increasing, restoring or replacing the functional activity of a diseased or damaged tissue or organ.

The artificial tissue of the invention can be used for partially or completely increasing, restoring or replacing the functional activity of any diseased or damaged tissue or organ of a living organism. The tissue or organ can be internal such as, without limitation, the urethra or the bladder, or external such as, without limitation, the cornea or the skin. In a preferred embodiment, the damaged tissues or organs are selected from the list comprising: skin, bladder, urethra, cornea, mucosa, conjunctiva, abdominal wall, conjunctiva, eardrum, pharynx, larynx, bowel, peritoneum, ligament, tendon, bone, meninx or vagina. The tissue or organ can be diseased or damaged as the result of a disorder, a lesion or a disease, for example, without limitation, an infectious, inflammatory, genetic or degenerative disease; a physical damage, such as a trauma or a surgical intervention, a chemical damage or a blood flow interruption.

A preferred embodiment of this third aspect relates to the use of the artificial tissue of the invention for partially or completely increasing, restoring or replacing the functional activity of skin. A more preferred embodiment relates to the use of the artificial tissue of the invention for partially or completely increasing, restoring or replacing the functional activity of diseased or damaged skin as the result of a disorder, lesion or disease selected from the list comprising: a wound, an ulcer, a burn, a benign or malignant neoplasm, an infection, a bruise, a trauma, a causticization or a congenital malformation.

A preferred embodiment of this third aspect relates to the use of the artificial tissue of the invention for partially or completely increasing, restoring or replacing the functional activity of a bladder. A more preferred embodiment relates to the use of the artificial tissue of the invention for partially or completely increasing, restoring or replacing the functional activity of a diseased or damaged bladder as the result of a disorder, lesion or disease selected from the list comprising: a benign or malignant neoplasm, an infection, a trauma, a congenital malformation (such as, without limitation, bladder exstrophy, a cloacal exstrophy or a contracted bladder), a neurogenic bladder, urinary incontinence, a bladder dysfunction, an infection or a bladder lithiasis.

A preferred embodiment of this third aspect relates to the use of the artificial tissue of the invention for partially or completely increasing, restoring or replacing the functional activity of a urethra. A more preferred embodiment relates to the use of the artificial tissue of the invention for partially or completely increasing, restoring or replacing the functional activity of a diseased or damaged urethra as the result of a disorder, lesion or disease selected from the list comprising: a benign or malignant neoplasm, an infection, a trauma, a congenital malformation (such as, without limitation, a hypospadias or an epispadias) or a stenosis.

A preferred embodiment of this third aspect relates to the use of the artificial tissue of the invention for partially or completely increasing, restoring or replacing the functional activity of a cornea. A more preferred embodiment relates to the use of the artificial tissue of the invention for partially or completely increasing, restoring or replacing the functional activity of a diseased or damaged cornea as the result of a disorder, lesion or disease selected from the list comprising: a corneal ulcer, a keratoconus, a keratoglobus, a descemetocele, a trauma, a causticization, a limbic impairment, an atrophic keratitis, a corneal dystrophy, a primary or secondary keratopathy, an infection, a leukoma, a bullous keratopathy, a corneal endothelial dysfunction or a benign or malignant neoplasm.

A preferred embodiment of this third aspect relates to the use of the artificial tissue of the invention for the production of a drug for partially or completely increasing, restoring or replacing the functional activity of a mucosa, preferably of an oral mucosa. An even more preferred embodiment relates to the use of the artificial tissue of the invention for partially or completely increasing, restoring or replacing the functional activity of a diseased or damaged oral mucosa as the result of a disorder, lesion or disease selected from the list comprising: a wound, an ulcer, a burn, a benign or malignant neoplasm, an infection, a bruise, a trauma, a causticization, a congenital malformation, a loss of substance or a periodontal disease. In a preferred embodiment, the tissue that is used for partially or completely increasing, restoring or replacing the functional activity of a mucosa is a tissue that has been subjected to a step (e2) of adding a protein between step (e) and step (f). In an even more preferred embodiment, said step is carried out by means of adding a composition comprising collagen to the material obtained in step (e), as indicated in detail above.

A **third aspect** of the present invention relates to the use of the artificial tissue of the invention for the production of a drug.

Said drug is a somatic cell therapy drug. *"Somatic cell therapy"* is understood as the use of living, autologous, allogeneic or xenogeneic somatic cells, the biological characteristic of which have been substantially altered as a result of their manipulation for obtaining a therapeutic, diagnostic or preventive effect through metabolic, pharmacological or immunological means. Included among somatic cell therapy drugs are, for example, without limitation: cells manipulated to modify their immunological, metabolic or other type of functional properties in qualitative and quantitative aspects; sorted, selected and manipulated cells which are subsequently subjected to a production process for the purpose of obtaining the end product; cells manipulated and combined with non-cellular components (for example, biological or inert matrices or medical devices) performing in principle the intended action in the finished product; autologous cell derivatives expressed *ex vivo (in vitro)* under specific culture conditions; or cells which are genetically modified or subjected to another type of manipulation to express homologous or non-homologous functional properties not expressed before.

A **third aspect** of the present invention relates to the use of the artificial tissue of the invention for the production of a drug for partially or completely increasing, restoring or replacing the functional activity of a tissue or organ. In a preferred embodiment, the damaged tissues or organs are selected from the list comprising: skin, bladder, urethra, cornea, mucosa, conjunctiva, abdominal wall, eardrum, pharynx, larynx, bowel, peritoneum, ligament, tendon, bone, meninx or vagina.

A preferred embodiment of this third aspect relates to the use of the artificial tissue of the invention for the production of a drug for partially or completely increasing, restoring or replacing the functional activity of a diseased or damaged tissue or organ as a consequence of an infectious, inflammatory, genetic or degenerative disease, a physical or chemical damage or a blood flow interruption.

A more preferred embodiment of this third aspect relates to the use of the artificial tissue of the invention for the production of a drug for partially or completely increasing, restoring or replacing the functional activity of skin. An even more preferred embodiment relates to the use of the artificial tissue of the invention for the production of a drug for partially or completely increasing, restoring or replacing the functional activity of diseased or damaged skin as the result of a disorder, lesion or disease selected from the list comprising: a wound, an ulcer, a burn, a benign or malignant neoplasm, an infection, a bruise, a trauma, a causticization or a congenital malformation.

A more preferred embodiment of this third aspect relates to the use of the artificial tissue of the invention for the production of a drug for partially or completely increasing, restoring or replacing the functional activity of a bladder. An even more preferred embodiment of this third aspect relates to the use of the artificial tissue of the invention for the production of a drug for partially or completely increasing, restoring or replacing the functional activity of a diseased or damaged bladder as the result of a disorder, lesion or disease selected from the list comprising: a benign or malignant neoplasm, an infection, a trauma, a congenital malformation (such as, without limitation, bladder exstrophy, a cloacal exstrophy or a contracted bladder), a neurogenic bladder, urinary incontinence, a bladder dysfunction, an infection or a bladder lithiasis.

A more preferred embodiment of this third aspect relates to the use of the artificial tissue of the invention for the production of a drug for partially or completely increasing, restoring or replacing the functional activity of a urethra. An even more preferred embodiment of this third aspect relates to the use of the artificial tissue of the invention for the production of a drug for partially or completely increasing, restoring or replacing the functional activity of a diseased or damaged urethra as the result of a disorder, lesion or disease selected from the list comprising: a benign or malignant neoplasm, an infection, a trauma, a congenital malformation (such as, without limitation, a hypospadias or an epispadias) or a stenosis.

A more preferred embodiment of this third aspect relates to the use of the artificial tissue of the invention for the production of a drug for partially or completely increasing, restoring or replacing the functional activity of a cornea. An even more preferred embodiment of this third aspect relates to the use of the artificial tissue of the invention for the production of a drug for partially or completely increasing, restoring or replacing the functional activity of a diseased or damaged cornea as the result of a disorder, lesion or disease selected from the list comprising: a corneal ulcer, a keratoconus, a keratoglobus, a descemetocele, a trauma, a causticization, a limbic impairment, an atrophic keratitis, a corneal dystrophy, a primary or secondary keratopathy, an infection, a leukoma, a bullous keratopathy, a corneal endothelial dysfunction or a benign or malignant neoplasm.

A more preferred embodiment of this third aspect relates to the use of the artificial tissue of the invention for the production of a drug for partially or completely increasing, restoring or replacing the functional activity of a mucosa, preferably an oral mucosa. An even more preferred embodiment relates to the use of the artificial tissue of the invention for the production of a drug for partially or completely increasing, restoring or replacing the functional activity of a diseased or damaged oral mucosa as the result of a disorder, lesion or disease selected from the list comprising: a wound, an ulcer, a burn, a benign or malignant neoplasm, an infection, a bruise, a trauma, a causticization, a congenital malformation, a loss of substance or a periodontal disease. In a preferred embodiment, the tissue that is used for the production of a drug for partially or completely increasing, restoring or replacing the functional activity of a mucosa is a tissue that has been subjected to a step (e2) of adding a protein between step (d) and step (f). In an even more preferred embodiment, said step is carried out by means of adding a composition comprising collagen to the material obtained in step (e), as indicated in detail above.

An **fourth aspect** of the invention relates to a pharmaceutical composition comprising the artificial tissue of the invention.

A preferred embodiment of this fourth aspect of the invention relates to a pharmaceutical composition comprising the artificial tissue of the invention for use in somatic cell therapy.

A more preferred embodiment of this fourth aspect of the invention relates to a pharmaceutical composition comprising the artificial tissue of the invention for partially or completely increasing, restoring or replacing the functional activity of a tissue or organ.

A preferred embodiment of this fourth aspect of the invention relates to a pharmaceutical composition comprising the artificial tissue of the invention for partially or completely increasing, restoring or replacing the functional activity of a diseased or damaged tissue or organ as a consequence of an infectious, inflammatory, genetic or degenerative disease, a physical or chemical damage or a blood flow interruption.

A more preferred embodiment of this fourth aspect of the invention relates to a pharmaceutical composition comprising the artificial tissue of the invention for partially or completely increasing, restoring or replacing the functional activity of skin. An even more preferred embodiment relates to a pharmaceutical composition comprising the artificial tissue of the invention for partially or completely increasing, restoring or replacing the functional activity of diseased or damaged skin as the result of a disorder, lesion or disease selected from the list comprising: a wound, an ulcer, a burn, a benign or malignant neoplasm, an infection, a bruise, a trauma, a causticization or a congenital malformation.

A more preferred embodiment of this fourth aspect of the invention relates to a pharmaceutical composition comprising the artificial tissue of the invention for partially or completely increasing, restoring or replacing the functional activity of a bladder. An even more preferred embodiment relates to a pharmaceutical composition comprising the artificial tissue of the invention for partially or completely increasing, restoring or replacing the functional activity of a diseased or damaged bladder as the result of a disorder, lesion or disease selected from the list comprising: a benign or malignant neoplasm, an infection, a trauma, a congenital malformation (such as, without limitation, bladder exstrophy, a cloacal exstrophy or a contracted bladder), a neurogenic bladder, urinary incontinence, a bladder dysfunction, an infection or a bladder lithiasis.

A more preferred embodiment of this fourth aspect of the invention relates to a pharmaceutical composition comprising the artificial tissue of the invention for partially or completely increasing, restoring or replacing the functional activity of a urethra. An even more preferred embodiment relates to a pharmaceutical composition comprising the artificial tissue of the invention for partially or completely increasing, restoring or replacing the functional activity of a diseased or damaged urethra as the result of a disorder, lesion or disease selected from the list comprising: a benign or malignant neoplasm, an infection, a trauma, a congenital malformation (such as, without limitation, a hypospadias or an epispadias) or a stenosis.

A more preferred embodiment of this fourth aspect of the invention relates to a pharmaceutical composition comprising the artificial tissue of the invention for partially or completely increasing, restoring or replacing the functional activity of a cornea. An even more preferred embodiment relates to a pharmaceutical composition comprising the artificial tissue of the invention for partially or completely increasing, restoring or replacing the functional activity of a diseased or damaged cornea as the result of a disorder, lesion or disease selected from the list comprising: a corneal ulcer, a keratoconus, a keratoglobus, a descemetocele, a trauma, a causticization, a limbic impairment, an atrophic keratitis, a corneal dystrophy, a primary or secondary keratopathy, an infection, a leukoma, a bullous keratopathy, a corneal endothelial dysfunction or a benign or malignant neoplasm.

A more preferred embodiment of this fourth aspect relates to a pharmaceutical composition comprising the artificial tissue of the invention for the production of a drug for partially or completely increasing, restoring or replacing the functional activity of a mucosa, preferably an oral mucosa. An even more preferred embodiment relates to pharmaceutical composition comprising the artificial tissue of the invention for partially or completely increasing, restoring or replacing the functional activity of a diseased or damaged oral mucosa as the result of a disorder, lesion or disease selected from the list comprising: a wound, an ulcer, a burn, a benign or malignant neoplasm, an infection, a bruise, a trauma, a causticization, a congenital malformation, a loss of substance or a periodontal disease. In a preferred embodiment, the pharmaceutical composition comprising the artificial tissue of the invention for the production of a drug for partially or completely increasing, restoring or replacing the functional activity of a mucosa or oral mucosa is a tissue that has been subjected to a step (e2) of adding a protein between step (e) and step (f). In an even more preferred embodiment, said step is carried out by means of adding a composition comprising collagen to the material obtained in step (e), as indicated in detail above.

In a preferred embodiment of this aspect of the invention, the pharmaceutical composition comprises the artificial tissue of the invention, and furthermore a pharmaceutically acceptable vehicle. In another preferred embodiment of this aspect of the invention, the pharmaceutical composition comprises the artificial tissue of the invention, and furthermore another active ingredient. In a preferred embodiment of this aspect of the invention, the pharmaceutical composition comprises the artificial tissue of the invention, and furthermore, together with a pharmaceutically acceptable vehicle, another active ingredient.

The pharmaceutical compositions of the present invention can be used in a treatment method in an isolated manner or together with other pharmaceutical compounds.

*KIT OR DEVICE OF THE INVENTION*

A **fifth aspect** of the invention relates to a kit or device, hereinafter *"kit or device of the invention,"* wherein the

EP 3 222 711 B1

kit or devices comprises:

a) the artificial tissue of the invention, and
b) an element capable of generating a magnetic field.

The magnetic field generating element can be, without limitation, a solenoid, a toroid, or a spiral, generating a magnetic field in certain regions of space when an electric current circulates through them. Magnetic field generating elements are known in the state of the art.

Preferably, the magnetic field applied is less than 500 kA/m, more preferably less than 100 kA/m, and even more preferably it will be comprised between 10 and 50 kA/m. In an even much more preferred embodiment, the magnetic field is between 16 and 48 kA/m.

Said magnetic field will preferably be generated in the region where the tissue containing magnetic particles is located and can be produced by means of coils through which a direct electric current circulates. It will preferably be a magnetic field that is uniform in space in the tissue containing magnetic particles.

In another preferred embodiment of this aspect of the invention, the time of application of the magnetic field ranges between 2 minutes and 1 hour, more preferably between 3 and 20 minutes, and even more preferably it is about 5 minutes.

In another preferred embodiment, the magnetic field is maintained while culturing the cells.

Throughout the description and the claims, the term *"comprises"* and variants thereof do not intend to exclude other technical features, supplements, components or steps. For those skilled in the art, other objects, advantages and features of the invention will be understood in part from the description and in part from the practice of the invention. The following examples and drawings are provided by way of illustration and are not meant to limit the present invention.

## EXAMPLES

Only the examples in which artificial tissues comprise multiple domain magnetic particles and in which a magnetic filed is applied at least between steps e and f, as defined in claim 1, are according to the claims.

The following specific examples provided in this patent document serve to illustrate the nature of the present invention. These examples are included only for illustrative purposes and must not be interpreted as limiting to the invention claimed herein. Therefore, the examples described below illustrate the invention without limiting the field of application thereof.

### Example 1. Preparation of a magnetic tissue

In this example, MagNP-OH particles (Nanomyp, Spain), which have a diameter of 100 nm and consist of a magnetite core ($\gamma$-$Fe_3O_4$) and are coated with a polymer matrix of methyl methacrylate-co.hydroxylethyl methacrylate-co-ethylene glycol dimethacrylate (MMA-co-HEMA-co-EGDMA), were used to prepare the biological tissues. The Mag-NP-OH nanoparticles were previously in an aqueous suspension stabilized with a surfactant. For the purpose of removing same, 5 washes were performed by means of: centrifuging at 15000 g for 30 minutes each, after that the supernatant was disposed of; bidistilled water was then added to redisperse the particles; after that the water was replaced with 70% ethanol and the nanoparticles were left in that solution for 12 hours for sterilization. Finally, the ethanol was removed and the nanoparticles were redispersed in an aqueous medium (Dulbecco's Modified Eagle's Medium, DMEM).

A mixture of fibrin and agarose was used as a biopolymer matrix and human gingival fibroblasts to generate an artificial oral mucosa. To that end, the following steps were followed.

i) 3.8 mL of human plasma were taken from blood donors (kindly provided by Dr. Fernandez-Montoya, Tissue Bank of Granada).

ii) 1,000,000 of human gingival fibroblasts dispersed in 0.625 mL of DMEM were added to said volume of plasma.

iii) 75 $\mu$L of tranexamic acid, which is an antifibrinolytic agent that prevents extracellular matrix degradation, were then added.

iv) The suspension of MagNP-OH particles in DMEM was then added until reaching a resulting concentration of about 2 mL of particles per 100 mL.

v) Next, 0.25 mL of agarose type VII in PBS (0.02 g/mL of concentration) were added to the mixture resulting from the previous step (iv), resulting in a final agarose concentration of 0.1%.

vi) 0.25 mL of a 2% $CaCl_2$ solution were added to the resulting mixture in step (v) for the purpose of activating fibrin polymerization.

vii) Finally, the mixture resulting from step (vi) was placed in Petri dishes and was kept at 37°C until gelling was completed. For the first 5 minutes of this gelling a vertical magnetic field was applied on the mixture by means of a solenoid connected to a direct current power source. Different field strengths, in a range of 0 to 48 kA/m (0, 16, 32 and 48 kA/m), were applied to the different samples.

The control samples were non-magnetic tissues prepared following the same protocol described above, with the exception that magnetic particles are not introduced, i.e., step (iv) described in the preceding paragraph was not performed. Different magnetic field strengths were also applied during gelling of the samples (0, 16, 32 and 48 kA/m), to analyze the effect on the different biological constituents of the tissues. Oral mucosa replacements produced by 8 different protocols (Table 1) were obtained. The density of all those replacements was about 1.1 g/mL.

Table 1. Different fibrin and agarose replacements produced.

| Magnetic field applied during gelling (kA/m) | 0 | | 16 | | 32 | | 48 | |
|---|---|---|---|---|---|---|---|---|
| Concentration of MagNP-OH particles (g per 100 mL) | 0 | 2 | 0 | 2 | 0 | 2 | 0 | 2 |
| Name of the sample | Ctrl-MF0 | M-MF0 | Ctrl-MF16 | M-MF16 | Ctrl-MF32 | M-MF32 | Ctrl-MF48 | M-MF48 |

The samples indicated in Table 1 will be used in Example 4 described below.

## Example 2. Analysis of magnetic particle biocompatibility in 2D cell culture systems

### Example 2a. Cell culture and treatment

In this example 20,000 human gingival fibroblasts cultured in 24-well plates with 500 μL of DMEM supplemented with 10% SBF and antibiotics/antimycotics were used. The cells were incubated at 37°C in a 5% carbon dioxide atmosphere for 24 hours. They were then washed twice in PBS and incubated in DMEM.

The MagNP-OH particles were dispersed in DMEM at a concentration of 5% (weight/vol) and 1% (weight/vol). These suspensions were placed in the plates in contact with the cells. The cells in contact with the MagNP- particles were incubated in an oven at 37°C and with 5% carbon dioxide for 24 hours. As a positive control (no toxicity), the cells were incubated under the same conditions without the presence of the MagNP-OH particles. As a negative control (100% toxicity), the cells were incubated in the same medium with the addition of 1% Triton X-100 (Sigma-Aldrich). Six independent experiments were performed, obtaining a total of 24 measurements per method and per condition.

### Example 2b. Morphostructural analysis by means of phase-contrast microscope.

To evaluate morphological damages caused by the presence of the MagNP-OH nanoparticles in human gingival fibroblasts, a phase-contrast microscope was used (Nikon Eclipse i90, Tokyo, Japan).

The results of this Example 2b show that the magnetic tissues have a macroscopic appearance similar to that of non-magnetic tissues, although with a darker color, in contrast with the greater transparency of the non-magnetic tissues.

The human gingival fibroblasts, used as a positive control, showed a typical elongated spindle shape (Figure 1). The cells exposed to the MagNp-OH nanoparticles maintained the characteristic spindle shape of the normal cells, and the nanoparticles can be seen in the extracellular space (Figures 1B and 1C). On the other hand, the fibroblasts used as a negative control showed a rounded shape (Figure 1D).

### Example 2c. WST-1 cell viability test

To evaluate MagNP-OH nanoparticle biocompatibility, the WST-1 test was used. To that end, the supernatant was removed from the wells and the cells were washed with PBS. A solution consisting of 10 μL of WST-1 reagent (Roche, Germany) and 100 μL of aqueous medium was then placed on said cells. The cells were incubated for 4 hours in that solution, and their viability was evaluated using a plate reader (Biochrom® Asys UVM340) in a wavelength range of 450-690 nm. The Mann-Whitney statistical test was used to compare the results and a p less than 0.05 was considered statistically significant.

The results of this Example 2c (see Figure 2) show that the positive control groups and the samples exposed to the MagNp-OH nanoparticles have a similar mitochondrial activity, there being no statistically significant differences between them ($p > 0.05$). Only the negative control showed very low mitochondrial activity with respect to the previous ones with statistically significant differences ($p = 0.000$).

## Example 2d. Free DNA quantification test

To identify the DNA released into the aqueous medium by the dead cells, the DNA quantification method was used. To that end, 10 $\mu$L of supernatant were taken from each well where the cells were cultured. Those 10 $\mu$L were then diluted in bidistilled water until obtaining a total volume of 100 $\mu$L and the DNA present in the samples was quantified using a spectrophotometer (Bio-Rad, ref. 170-2510, Hercules, California, USA) in a wavelength range of 260-280 nm. The conversion factor was 1 = 50 $\mu$g/mL. The Mann-Whitney statistical test was used to compare the results and a p value less than 0.05 was considered statistically significant.

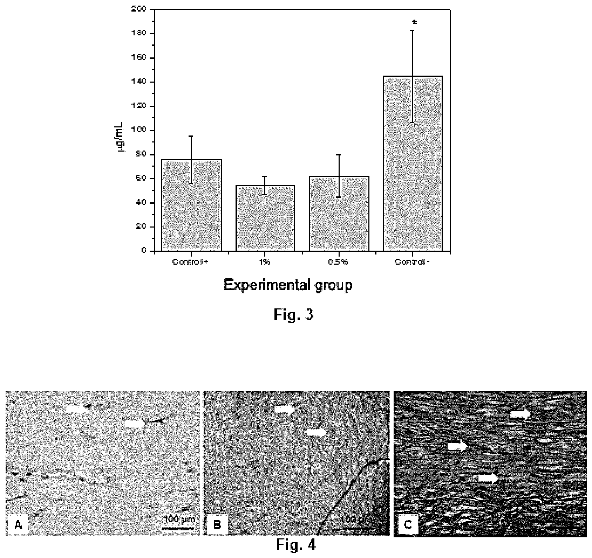

The results of the identification of free DNA fragments in the cellular aqueous medium showed that the samples exposed to the MagNP-OH nanoparticles and the positive control form a homogenous sub-group, there being no significant differences between them ($p > 0.05$) (Figure 3), in turn being distinguished from the negative control ($p = 0.002$) which had significantly higher free DNA values.

## Example 3. Analysis of magnetic particle biocompatibility in a 3D system

## Example 3a. Morphostructural analysis by means of light and scanning electron microscopy

2) MagNP-OH particle biocompatibility was analyzed in this example in a three-dimensional tissue system. For this purpose, 24 hours after preparing the artificial tissues that contained human cells, said tissues were fixed in formalin, included in paraffin and 5 $\mu$m sections were obtained. After deparaffinization of the samples, the tissues were stained with hematoxylin and eosin (H&E) and were observed by light microscopy to analyze the biological structure thereof. The samples were also characterized using scanning electron microscopy (SEM) by fixing them in glutaraldehyde. The structure of the biomaterial and the cell shapes were analyzed in both cases.

3) The results of the macroscopic analysis (light microscopy) of the particles immersed in the three-dimensional tissues systems showed that the magnetic tissues had an appearance similar to that of the non-magnetic tissues, although they had a dark color, in contrast with the more transparent color of the non-magnetic constructs.

4) For the group gelled in the absence of a magnetic field (Ctrl-MFO), the analysis showed spindle-shaped and star-shaped cells, compatible with normality (Figure 4A). Furthermore, the cells were distributed throughout all the fibrin-agarose biomaterials, having a normal appearance, similar to a scaffold. Similar conclusions were obtained from observing the control groups gelled in the presence of an applied magnetic field, although they are not herein presented for the sake of brevity.

5) With respect to the samples that contained magnetic particles, different cases could be distinguished. The sample with particles, gelled in the absence of an applied magnetic field (M-MF0), showed an isotropic and homogenous fibrillar pattern, with randomly distributed particles (Figure 4B). On the other hand, it was observed that the samples with particles, gelled under the application of a magnetic field (M-MF16, M-MF32 and M-MF48), had a pattern that consisted of filamentous structures aligned in a specific direction, regardless of the strength of the applied magnetic field - see the image of sample M-MF32 (Figure 4C) as an example.

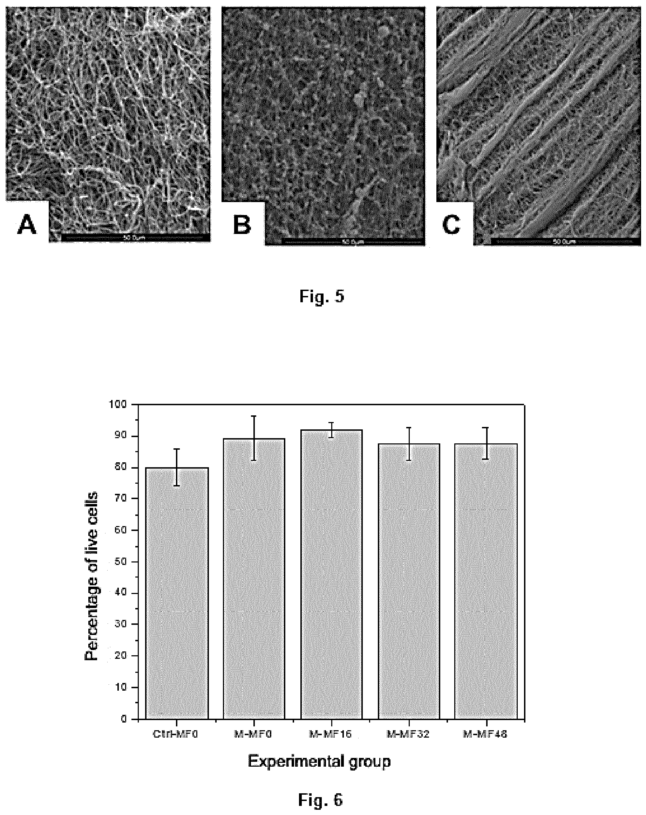

6) The scanning electron microscopy images show that the control samples had an isotropic and homogenous fibrillar pattern (see sample Ctrl-MFO - Figure 5A). The application of a magnetic field during gelling of the control samples did not cause any significant change in its microscopic morphology; samples Ctrl-MF12 and Ctrl-MF48 had a pattern similar to Ctrl-MFO (not included herein for the sake of brevity).

7) Adding the MagNP-OH nanoparticles caused some changes at the microscopic level with a modification of the fibrillar pattern, even in the absence of the application of a magnetic field during gelling. Nevertheless, in this case the biomaterial conserved its homogenous morphology, some particles and particle clusters were distributed homogenously throughout the entire fibrin scaffold, interrupting its order at the mesoscopic level (Figure 5B). When a magnetic field was applied during gelling, the fibrin scaffold had an anisotropic pattern at the microscopic level. This is characterized by the existence of a privileged direction with the presence of thick bands that contained closely packed fibrin fibers that are aligned and braided in the direction of the bands. Furthermore, there is an increase in the spaces in which there is a reticular isotropic distribution between the bands, with a lower fiber concentration - see as an example the scanning microscopy image for sample M-MF48 (Figure 5C). It is important to mention that the thick bands became more evident when the strength of the magnetic field applied during gelling is increased.

### Example 3b. Cell viability analysis

Cell viability was analyzed by means of the activity of an intracellular sterase and the cytoplasmic and nuclear membrane integrity using the LIVE/DEAD™ test (Life Technologies, Carlsbad, CA). To that end, a tissue sample of 3 mm in diameter was taken 24 hours after preparation, the supernatant was removed and washed with PBS. Next, the samples were incubated in a LIVE/DEAD™ solution for 15 minutes, as indicated by the manufacturer, and it was then washed in PBS. The samples were observed by fluorescence microscopy and the images were processed by means of ImageJ software to quantify the number of live cells (stained green) and dead cells (stained red).

Cell viability was also evaluated by analyzing nuclear membrane integrity by means of quantifying the DNA released into the aqueous medium. For this purpose, the supernatant was taken from each sample; 10 $\mu$L aliquots were then extracted; these aliquots were diluted in 100 $\mu$L of bidistilled water (Ambion-Life Technologies, Austin, TX). The released DNA was quantified by means of a spectrophotometer (SmartSpecTM plus BIO-RAD, Hercules, CA) in a wavelength range of 260-280 nm.

The results of these Live & Dead cell viability assays showed that the number of viable cells was above 80% for all the experimental groups, without any significant differences (p > 0.05) between the groups (Figure 6).

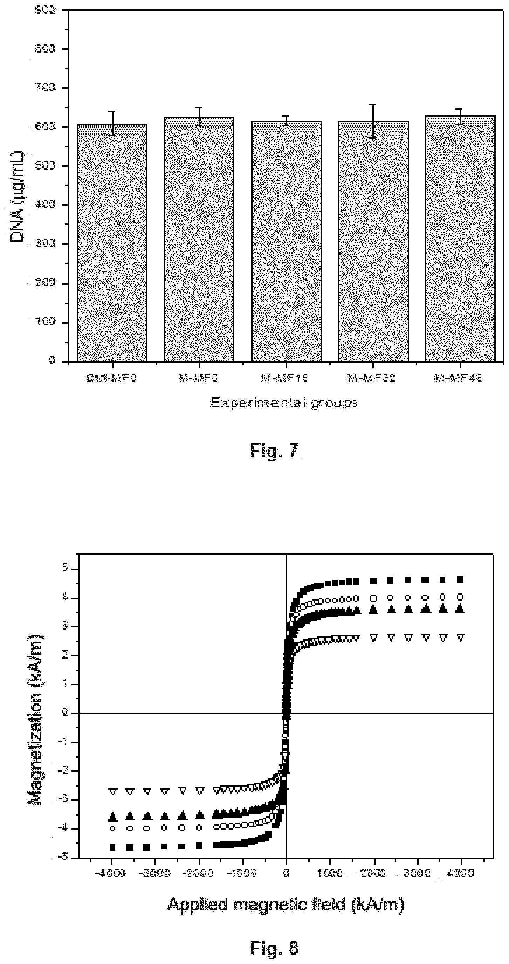

Similarly, no significant difference (p>0.05) was observed between the free DNA values in the constructs of the different experimental groups (Figure 7).

### Example 4. Magnetic and mechanical properties

Magnetization, M, of the particles made up of the material MagNP-OH, in dry powder form, was measured according to the applied field strength, H, in a Squid Quantum Design MPMS XL magnetometer. Similarly, the magnetization curve (M as a function of H) of the tissues, with the moisture they contain, was obtained 24 hours after preparation. In both cases, the measurement temperature was 37°C.

The results of the magnetization curve (not shown herein for the sake of brevity) show that the MagNP-OH particles show the typical behavior of a ferromagnetic material. The saturation magnetization of these particles, obtained by means of adjusting magnetization data to the Frohlich-Kennely law [Jiles 1991], was 161 $\pm$ 7 kA/m.

Similarly, the magnetic tissues are ferromagnetic, though with a much lower saturation magnetization value than the magnetic particles do (Figure 8). The differences in the magnetization of the different magnetic tissue samples can be attributed to their different MagNP-OH particle content. In fact, the concentration of magnetic particles in each tissue can be obtained from the saturation magnetization values of the tissue and of the dry particles using the law of mixtures [Rosensweig 1985]. The following magnetic particle concentration values (expressed as a volume fraction occupied by same $\phi$ vol.) were thereby obtained in the different tissue samples: M-MF0: $\phi$ = (2.9 $\pm$ 0.3) vol.%; M-MF16: $\phi$ = (2.5 $\pm$ 0.3) vol.%; M-MF32: $\phi$ = (1.66 $\pm$ 0.16) vol.%; M-MF48: $\phi$ = (2.22 $\pm$ 0.22) vol.%. The control (non-magnetic) tissue obviously does not show ferromagnetic behavior.

The mechanical properties of the tissue samples were measured, 24 hours after preparation at 37°C, with a Haake brand MARS III controlled stress rheometer (Thermo Fisher Scientific, USA). The measurement geometry chosen in the rheometer was the parallel plate geometry. These are discs with a diameter of 3.5 cm in which the surface in contact with the sample had the roughness required to prevent the sliding phenomenon on walls. The tissue samples were gelled in Petri dishes with the same diameter of 3.5 cm as the plates of the rheometer.

The method followed is described below. First, the tissue sample was located on the lower plate of the rheometer and was compressed with the upper rotor plate thereof until reaching a normal force of 5 N on the rotor plate. The distance between the two plates of the measurement system for which it reached said normal force value varied slightly from one sample to the next, but in all cases it was at about 300 $\mu$m. The measurements were taken both in the absence and in the presence of a magnetic field. To that end, a coil was provided surrounding the measurement plates the axis of symmetry of which coincided with the axis of rotation of the rotating upper plate of the rheometer. In the rheological measurements under a magnetic field, said field was applied 1 minute before initiating the measurements and was applied the entire time the experiment lasted.

Three different types of rheological assays were performed.

(i) Oscillometry with amplitude scanning. In this type of assay, the sample was subjected to a shear strain that varied in a sinusoidal manner; frequency was kept constant (1 Hz) and the amplitude of the strain ($\gamma_0$) was increased as the corresponding oscillatory stress values were measured. The modulus of elasticity (G') and modulus of viscosity (G") according to $\gamma_0$ were calculated from these measurements. The range of values of $\gamma_0$ in which the material has a viscoelastic linear behavior (or viscoelastic linear region, VLR) is thereby obtained.

(ii) Oscillometry with frequency scanning. In this case, the sinusoidal strain that is applied has a constant amplitude $\gamma_0$ (within the VLR range) and the oscillation frequency is varied, the corresponding oscillograms, i.e., G' and G" according to the frequency, being obtained. In these two oscillometric assays, each value of the strain amplitude (assay i) or frequency (assay ii) magnitudes were maintained for 5 cycles. Only the values obtained in the last 3 cycles were taken into account to prevent transient effects.

(iii) Steady state assay. In this case, the sample was maintained with a constant shear strain for 10 seconds and the resulting shear stress was measured. This process was repeated for increasing strain values (strain ramp) until reaching the non-linear stress-strain dependence area. The modulus of rigidity of the samples was obtained from the initial linear part of the stress-strain curves. All the mentioned assays were conducted with different aliquots of each type of sample. The results that are described below always correspond to the mean values obtained with at least 3 different aliquots.

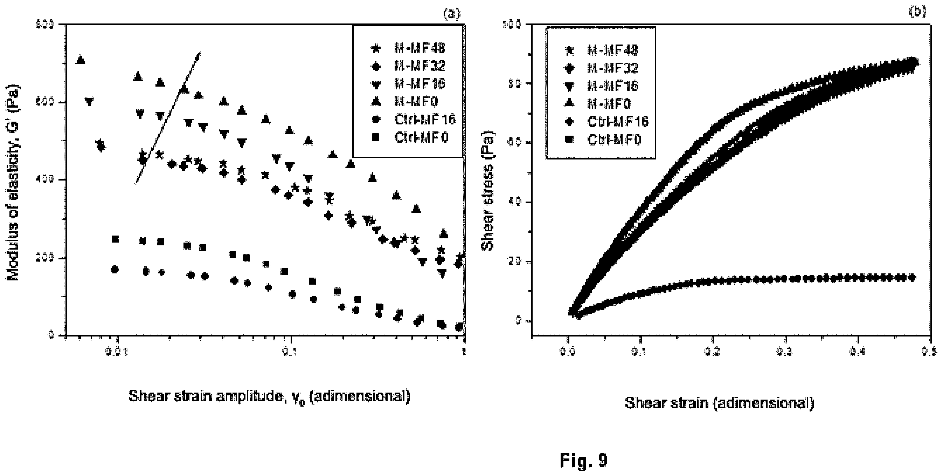

Only the values for modulus of elasticity G' as a function of the amplitude of the strain $\gamma_0$ at a frequency of 1 Hz (Figure 9A) are shown here. The results of the steady state assay (stress vs. strain, Figure 9B) are also shown. In the oscillograms obtained for other frequencies other than 1 Hz, trends similar to the trend shown in Figure 9A were obtained. The same can be said (the same trends) with respect to the values of the modulus of viscosity obtained in the oscillograms with amplitude scanning or in the oscillograms with frequency scanning.

In the absence of a magnetic field, much higher values (up to 4 times higher) of the modulus of elasticity (G') are observed in the samples containing magnetic particles than in those that do not (Figure 9A). Something similar can be said with respect to the shear stress values, which are up to 5 times higher for a given strain value, in the case of the magnetic samples (Figure 9B). The deviation between the values obtained in the control (non-magnetic) samples was small. In the case of the magnetic samples, the deviations in the G' values (oscillometric assays) for different aliquots of one and the same type of sample reach up to 40%. However, the values obtained in the shear stress (steady-state tests) in the aliquots of the magnetic samples are statistically coincident, with the exception of the magnetic sample in which gelling was performed in the absence of an applied magnetic field (M-MF0). With respect to the dependence between G' and $\gamma_0$, it is observed that in all the cases there is an initial pseudo plateau for the lowest $\gamma_0$ values followed by a drop in mean and high $\gamma_0$ values. The initial pseudo plateau corresponds to the viscoelastic linear region (VLR), whereas the rest of the curve corresponds to the viscoelastic non-linear behavior region. The G' value in the VLR is usually taken as a parameter which characterizes the rigidity of the material, wherein said rigidity is greater the greater G' is. In the case of the shear stress vs. strain curves, it is observed that there is a linear relationship for low strain values, i.e., stress and strain are proportional (elastic behavior) the modulus of rigidity, G, being the proportionality constant. For higher strain values, said elastic behavior is not maintained, such that stress increases more slowly the higher the strain (plastic behavior). On the other hand, the G values for the magnetic samples can be up to three times higher than those of the non-magnetic samples (see Table 2). This also demonstrates that the magnetic samples are more rigid than the non-magnetic samples given that G quantifies the rigidity of an elastic material. Furthermore, it must be pointed out that the elastic behavior (stress-strain linearity) in the magnetic samples is maintained up to much higher strain values (Figure 9B).

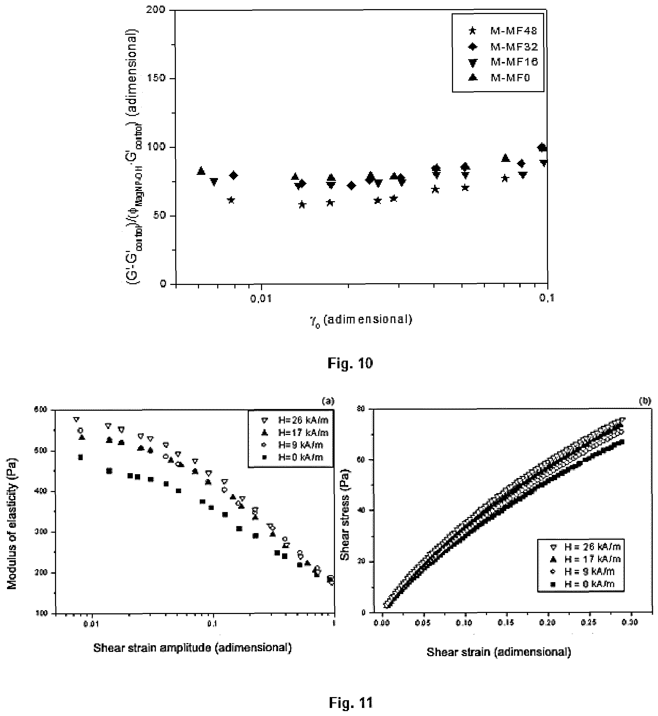

The G' values (pertaining to the VLR) of the different magnetic samples are now compared. In this case, it must be taken into account that the different samples contain a volume fraction ($\phi$) of different magnetic particles. Therefore, the increase in G' in each magnetic sample with respect to its value in the non-magnetic control tissue (G'$_{control}$) has been calculated to perform this analysis and said increase has been normalized by dividing by the magnetic particle volume fraction ($\phi_{MagNP-OH}$) and by the G'$_{control}$ value. Therefore, the adimensional parameter $\phi_{MagNP-OH} \cdot G'_{control}$ will be used. The values of this parameter have been depicted as a function of the amplitude of the strain $\gamma_0$ in Figure 10. In this figure it can be seen that the data for the different magnetic tissues virtually overlap in a single master curve.

As previously mentioned, the modulus of rigidity, G, of the tissues can be obtained from the slope of the initial linear area of the curves depicted in Figure 9B. The adimensional modulus of rigidity $\dfrac{G - G_{control}}{\phi_{MagNP-OH} \cdot G_{control}}$ can be defined similarly to that indicated for the modulus of elasticity G'. The corresponding results are shown in Table 2. Again, the adimensional moduli of the different samples overlap, and are not significantly different taking into account the standard deviations of the values shown in Table 2.

Table 2. Normalized modulus of rigidity $\dfrac{G - G_{control}}{\phi_{MagNP-OH} \cdot G_{control}}$ of the magnetic tissue samples in the absence of a magnetic field. The mean modulus of rigidity value of the non-magnetic sample is: $G_{control} = (101 \pm 10)$ Pa.

| Sample | M-MF0 | M-MF16 | M-MF32 | M-MF48 |
|---|---|---|---|---|
| Normalized modulus of rigidity: $\dfrac{G - G_{control}}{\phi_{MagNP-OH} \cdot G_{control}}$ (adimensional) | 81 ± 21 | 49 ± 16 | 74 ± 22 | 58 ± 17 |

With respect to the possible influence of the strength of the magnetic field applied during the rheological measurements, no effect whatsoever was observed in the case of the non-magnetic control sample. In the case of sample M-MF0, there were small differences in the obtained G' values (not shown for the sake of brevity). However, in this same sample M-MF0 there was a significant effect on the values obtained for the shear stress measured in the steady-state tests: in fact, a clear trend to increase the stress necessary to reach a specific strain value was observed when the strength of the magnetic field was increased (these values are not shown either). In samples M-MF32 and M-MF48, a significant effect of the magnetic field on both the G' values (oscillometry) and on the shear stress values (steady state) that are shown in Figure 11A was also observed. The shape of the G'-$\gamma_0$ curves obtained for the different field strengths applied is similar, although on average G' tends to increase as the field strength increases. The same can be said for the stress-strain curves (Figure 11B). The moduli of rigidity G, which show a clear trend to increase with the magnetic field in all samples (Table 3), were obtained again from the data depicted in this last figure.

Table 3. Effect of the magnetic field on the modulus of rigidity of the magnetic tissues.

| Strength of the magnetic field applied during the measurements (kA/m) | 0 | 9 | 17 | 26 |
|---|---|---|---|---|
| Sample M-MF0 | 338 ± 3 | 363 ± 3 | 369 ± 4 | 370 ± 4 |
| Sample M-MF16 | 224 ± 3 | 237 ± 3 | 246 ± 3 | 253 ± 3 |
| Sample M-MF32 | 225.8 ± 2.2 | 243.6 ± 2.1 | 239.6 ± 1.9 | 245 ± 1.9 |
| Sample M-MF48 | 230 ± 3 | 237 ± 3 | 252 ± 3 | 254 ± 3 |

**Claims**

1. An artificial tissue obtained or obtainable by a method which comprises:

   a) adding a composition comprising fibrinogen to a sample of isolated cells,
   b) adding an anti-fibrinolytic agent to the product resulting from step (a),
   c) adding at least a coagulation factor, a source of calcium, thrombin, or any combination of the above to the product resulting from step (b),
   d) adding a multiple magnetic domain particle with a mean diameter greater than 25 nm, preferably coated with a polymeric material, wherein the polymeric material is preferably a polymer matrix of methyl methacrylate-co.hydroxylethyl methacrylate-co-ethylene glycol dimethacrylate (MMA-co-HEMA-co-EGDMA) to the product resulting from step (c),
   e) adding a composition of a polysaccharide to the product resulting from step (d),
   f) culturing isolated cells in or on the product resulting from step (e), and
   g) inducing the nanostructuring of the product resulting from step (f), by inducing a structural modification consisting of generating bonds having a size of less than one micron between the fibrin fibers and between fibrin fibers and the polysaccharide molecules, wherein a magnetic field is applied at least between steps (e) and (f).

2. The artificial tissue according to claim 1, wherein the cells of step (a) are fibroblasts or keratocytes.

3. The artificial tissue according to any of claims 1 to 2, wherein the induction of the nanostructuring of step (g) comprises the dehydration and/or mechanical compression of the product resulting from step (f).

4. The artificial tissue according to any of claims 1 to 3, where between step (f) and step (g) there is an additional step in which the product resulting from step (f) is exposed to air.

5. The artificial tissue according to any of the precedent claims, wherein the magnetic domain particle are magnetite particles; or the particles consist of a magnetite core ($\gamma$-Fe$_3$O$_4$) and are coated with a polymer matrix of methyl methacrylate-co-hydroxylethyl methacrylate-co-ethylene glycol dimethacrylate (MMA-co-HEMA-co-EGDMA).

6. The artificial tissue according to any of the precedent claims, wherein the multiple magnetic domain particles have a mean diameter of about 100 nm.

7. The artificial tissue according to any of the precedent claims, wherein the artificial tissue or organ is selected from the list comprising: skin, bladder, urethra, cornea, mucosa, conjunctiva, abdominal wall, conjunctiva, eardrum, pharynx, larynx, bowel, peritoneum, ligament, tendon, bone, meninx or vagina.

8. The artificial tissue according to anyone of claims 1 to 7, for use in medicine.

9. A pharmaceutical composition comprising the artificial tissue according to anyone of claims 1 to 7.

10. The pharmaceutical composition according to claim 9, wherein the pharmaceutical composition further comprises a pharmaceutically acceptable vehicle and optionally a further active ingredient.

**Patentansprüche**

1. Künstliches Gewebe, welches mittels eines Verfahrens erhalten wird oder erhältlich ist, welches Folgendes umfasst:

a) das Hinzufügen einer Zusammensetzung umfassend Fibrinogen zu einer Probe von isolierten Zellen,
b) das Hinzufügen eines Antifibrinolytikum zum Produkt, welches aus Schritt (a) resultiert,
c) das Hinzufügen mindestens eines Gerinnungsfaktors, einer Quelle von Calcium, Thrombin oder jeder Kombination der Vorstehenden zum Produkt, welches aus Schritt (b) resultiert,
d) das Hinzufügen eines Teilchens mit mehrfachen magnetischen Domänen mit einem mittleren Durchmesser größer als 25 nm, vorzugsweise beschichtet mit einem Polymermaterial, wobei das Polymermaterial vorzugsweise eine Polymermatrix aus Methylmethacrylat-co-Hydroxylethylmethacrylat-co-Ethylenglycoldimethacrylat (MMA-co-HEMA-co-EGDMA) ist, zum Produkt, welches aus Schritt (c) resultiert,
e) das Hinzufügen einer Zusammensetzung eines Polysaccharids zum Produkt, welches aus Schritt (d) resultiert,
f) das Kultivieren von isolierten Zellen in oder auf dem Produkt, welches aus Schritt (e) resultiert, und
g) das Induzieren der Nanostrukturierung des Produkts, welches aus Schritt (f) resultiert, indem eine strukturelle Modifikation bestehend aus dem Erzeugen von Bindungen mit einer Größe kleiner als ein Mikrometer zwischen den Fibrinfasern und zwischen Fibrinfasern und den Polysaccharidmolekülen induziert wird,

wobei ein Magnetfeld mindestens zwischen den Schritten (e) und (f) angewendet wird.

2. Künstliches Gewebe nach Anspruch 1, wobei die Zellen aus Schritt (a) Fibroblasten oder Keratozyten sind.

3. Künstliches Gewebe nach einem der Ansprüche 1 bis 2, wobei die Induktion der Nanostrukturierung aus Schritt (g) das Dehydrieren und/oder das mechanische Zusammendrücken des Produkts, welches aus Schritt (f) resultiert, umfasst.

4. Künstliches Gewebe nach einem der Ansprüche 1 bis 3, wobei es zwischen Schritt (f) und Schritt (g) einen zusätzlichen Schritt gibt, in welchem das Produkt, welches aus Schritt (f) resultiert, der Luft ausgesetzt wird.

5. Künstliches Gewebe nach einem der vorhergehenden Ansprüche, wobei das Teilchen mit magnetischen Domänen Magnetitteilchen sind; oder die Teilchen aus einem Magnetitkern ($\gamma$-Fe$_3$O$_4$) bestehen und mit einer Polymermatrix aus Methylmethacrylat-co-Hydroxylethylmethacrylat-co-Ethylenglycoldimethacrylat (MMA-co-HEMA-co-EGDMA) beschichtet sind.

**6.** Künstliches Gewebe nach einem der vorhergehenden Ansprüche, wobei die Teilchen mit mehrfachen magnetischen Domänen einen mittleren Durchmesser von etwa 100 nm aufweisen.

**7.** Künstliches Gewebe nach einem der vorhergehenden Ansprüche, wobei das künstliche Gewebe oder das Organ aus der Liste ausgewählt wird, welche Folgendes umfasst: Haut, Harnblase, Harnröhre, Hornhaut, Schleimhaut, Bindehaut, Bauchwand, Bindehaut, Trommelfell, Rachen, Kehlkopf, Darm, Bauchfell, Ligament, Sehne, Knochen, Hirnhaut oder Scheide.

**8.** Künstliches Gewebe nach einem der Ansprüche 1 bis 7, für dessen Verwendung in Medizin.

**9.** Pharmazeutische Zusammensetzung umfassend das künstliche Gewebe nach einem der Ansprüche 1 bis 7.

**10.** Pharmazeutische Zusammensetzung nach Anspruch 9, wobei die pharmazeutische Zusammensetzung zusätzlich einen pharmazeutisch akzeptablen Träger und wahlweise einen zusätzlichen Wirkstoff umfasst.


**Revendications**

**1.** Tissu artificiel obtenu ou susceptible d'être obtenu par un procédé qui comprend :

a) l'addition d'une composition comprenant du fibrinogène à un échantillon de cellules isolées,
b) l'addition d'un agent anti-fibrinolytique au produit résultant de l'étape (a),
c) l'addition d'au moins un facteur de coagulation, une source de calcium, thrombine, ou toute combinaison de ce qui précède au produit résultant de l'étape (b),
d) l'addition d'une particule magnétique multi-domaine avec un diamètre moyen supérieur à 25 nm, de préférence revêtue d'un matériau polymérique, dans lequel le matériau polymérique est de préférence une matrice de polymère de méthacrylate de méthyle-co-méthacrylate d'hydroxyéthyle-co-diméthacrylate d'éthylène glycol (MMA-co-HEMA-co-EGDMA) au produit résultant de l'étape (c),
e) l'addition d'une composition d'un polysaccharide au produit résultant de l'étape (d),
f) la culture de cellules isolées dans ou sur le produit résultant de l'étape (e), et
g) l'induction de la nanostructuration du produit résultant de l'étape (f), en induisant une modification structurale consistant à générer des liaisons ayants une dimension inférieure à un micron entre les fibres de fibrine et entre les fibres de fibrine et les molécules de polysaccharide,

dans lequel un champ magnétique est appliqué au moins entre les étapes (e) et (f).

**2.** Tissu artificiel selon la revendication 1, dans lequel les cellules de l'étape (a) sont des fibroblastes ou des kératocytes.

**3.** Tissu artificiel selon l'une quelconque des revendications 1 à 2, dans lequel l'induction de la nanostructuration de l'étape (g) comprend la déshydratation et/ou la compression mécanique du produit résultant de l'étape (f).

**4.** Tissu artificiel selon l'une quelconque des revendications 1 à 3, dans lequel entre l'étape (f) et l'étape (g) il y a une étape supplémentaire dans laquelle le produit résultant de l'étape (f) est exposé à l'air.

**5.** Tissu artificiel selon l'une quelconque des revendications précédentes, dans lequel les particules du domaine magnétique sont des particules de magnétite ; ou les particules consistent en un noyau de magnétite ($\gamma$-Fe$_3$O$_4$) et sont revêtues d'une matrice de polymère de méthacrylate de méthyle-co-méthacrylate d'hydroxyéthyle-co-diméthacrylate d'éthylène glycol (MMA-co-HEMA-co-EGDMA).

**6.** Tissu artificiel selon l'une quelconque des revendications précédentes, dans lequel les particules magnétiques multi-domaine ont un diamètre moyen d'environ 100 nm.

**7.** Tissu artificiel selon l'une quelconque des revendications précédentes, dans lequel le tissu ou organe artificiel est choisi parmi la liste comprenant : la peau, la vessie, l'urètre, la cornée, la muqueuse, la conjonctive, la paroi abdominale, la conjonctive, le tympan, le pharynx, le larynx, l'intestin, le péritoine, le ligament, le tendon, l'os, la méninge ou le vagin.

**8.** Tissu artificiel selon l'une quelconque des revendications 1 à 7, pour son utilisation en médecine.

9. Composition pharmaceutique comprenant le tissu artificiel selon l'une quelconque des revendications 1 à 7.

10. Composition pharmaceutique selon la revendication 9, dans laquelle la composition pharmaceutique comprend en outre un véhicule pharmaceutiquement acceptable et optionnellement un autre principe actif.

**Fig. 1**

**Fig. 2**

**Fig. 3**

**Fig. 4**

Fig. 5

Fig. 6

Fig. 7

Fig. 8

Fig. 9

Fig. 10

Fig. 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 2471902 A

### Non-patent literature cited in the description

- **LANGER R ; VACANTI JP.** Tissue engineering. *Science,* 1993, vol. 260 (5110), 920-926
- **BADDOUR JA et al.** Birth Defects Res. *Part C-Embryo Today-Rev.,* 2012, vol. 96, 1-29
- **KHADEMHOSSEINI A et al.** *Proc. Natl. Acad. Sci. U.S.A.,* 2006, vol. 103, 2480-2487
- **HUTMACHER DW.** *Biomaterials,* 2000, vol. 21, 2529-2543
- **HAN L et al.** *Ann. Rev. Mater. Res.,* 2011, vol. 41, 133-168
- **PABBRUWE MB et al.** *Biomaterials,* 2009, vol. 30, 4277-4286
- **KON E et al.** *Injury,* 2010, vol. 41, 693-701
- **BOCK N et al.** *Acta Biomater.,* 2010, vol. 6, 786-796
- **DAS B et al.** *Biomed. Mater.,* 2013, vol. 8, 035003
- **HU H et al.** *RSC Adv.,* 2013, vol. 3, 879-886
- **LI et al.** *Adv. Funct. Mater.,* 2013, vol. 23, 660-672
- **ZHU et al.** *J. Mater. Chem. B,* 2013, vol. 1, 1279-1288